# EUROPÄISCHE PATENTSCHRIFT

(11) **EP 2 262 822 B1**
(45) Veröffentlichungstag und Bekanntmachung des Hinweises auf die Patenterteilung: **28.09.2011**
(21) Anmeldenummer: 09732936.1
(22) Anmeldetag: 06.04.2009
(51) Int. Cl.: C07H 15/04, A61K 8/60

(54) **ALKYL- UND/ODER ALKENYLETHER VON ALKYL- UND/ODER ALKENYL(POLY)GLYCOSIDEN UND IHRE VERWENDUNG**
ALKYL AND/OR ALKENYL ETHER OF AKLYL AND/OR ALKENYL(POLY)GLYCOSIDES AND USE THEREOF
ETHERS ALKYLIQUES ET/OU ALCÉNYLIQUES D'ALKYL- ET/OU D'ALCÉNYL(POLY)GLYCOSIDES ET LEUR UTILISATION

(30) Priorität: 14.04.2008 EP 08007291; 07.11.2008 EP 08019490
(43) Veröffentlichungstag der Anmeldung: 22.12.2010
(73) Patentinhaber: Cognis IP Management GmbH, 40589 Düsseldorf (DE)
(72) Erfinder: BEHLER, Ansgar, 46240 Bottrop (DE); ANSMANN, Achim, 40699 Erkrath (DE); WEICHOLD, Catherine, 52072 Aachen (DE); CLASEN, Frank, 40723 Hilden (DE); WICK, Anja, 40723 Hilden (DE); MAHNKE, Eike Ulf, 42553 Velbert (DE); KLOTZ , Bjoern, 40699 Erkrath (DE); NEUMANN, Carsten, 40476 Düsseldorf (DE); HLOUCHA, Matthias, 50677 Köln (DE); KAWA, Rolf, 40789 Monheim (DE); SCHULTE, Petra, 50733 Köln (DE)
(86) Internationale Anmeldenummer: PCT/EP2009/002511
(87) Internationale Veröffentlichungsnummer: WO 2009/127339

(56) Entgegenhaltungen:
- EP-A- 0 304 627
- EP-A- 0 364 852
- EP-A- 1 502 644
- DE-A1- 4 439 091
- DE-A1- 19 726 785

## Beschreibung

### Gebiet der Erfindung

Die Erfindung befindet sich auf dem Gebiet der kosmetischen und/oder pharmazeutischen Zubereitungen und betrifft Alkyl- und/oder Alkenylether von Alkyl- und/oder Alkenyl(poly)glycosiden, Gemische daraus, sowie ihre Verwendung, insbesondere in Nanoemulsionen. Die Erfindung betrifft weiterhin neue Nanoemulsionen sowie deren Verwendung. Die Erfindung betriff weiterhin neue Lösungsvermittler (Solubilisatoren) mit einem erhöhten Lösungsvermögen insbesondere für fettlösliche Wirkstoffe und UV-Lichtschutzfilter.

### Stand der Technik

Unter Nanoemulsionen werden üblicherweise Emulsionen verstanden, deren Teilchen- bzw. Tröpfchengröße bei unter 1000 nm liegt. Insbesondere versteht man unter Nanoemulsionen solche mit einer durchschnittlichen Teilchengröße von etwa 5 bis 500 nm. Nanoemulsionen finden wegen ihrer günstigen Eigenschaften häufig Anwendung in kosmetischen und pharmazeutischen Zubereitungen. Vorteilhaft sind vor allem die Phasenstabilität auch bei geringen Viskositäten sowie die gegenüber herkömmlichen Emulsionen deutlich erhöhte Resorptionsrate für aktive Wirkstoffe, die mit der Emulsion zum Beispiel auf Haut oder Haar aufgebracht werden.

Stabile Nanoemulsionen werden bisher fast ausschließlich nach der Phaseninversionsmethode (PIT-Verfahren) erhalten. Hierbei können jedoch nur ethoxylierte Emulgatoren eingesetzt werden. Diese sind jedoch oft hautreizend und von daher nachteilig.

Aus der DE 4439091 A1 sind oberflächenaktive Mittel, darunter auch kosmetische Mittel bekannt, die als Solubilisatoren kurzkettige Alkyloligoglycoside enthalten. Die DE 19726785 A1 beschreibt kosmetische und dermatologische Lichtschutzformulierungen, die bestimmte Triazinderivate enthalten und weiterhin ein Alkyloligoglycosid mit einem Glykolisierungsgrad von bis zu 2, das als Lösungsmittel oder Lösungsvermittler für die Triazinderivate eingesetzt wird.

Die Aufgabe der vorliegenden Erfindung bestand darin, Nanoemulsionen bereit zu stellen, welche möglichst ohne ethoxylierte Emulgatoren stabil hergestellt werden können. Darüber hinaus war es von Interesse sensorisch verbesserte Nanoemulsionen bereit zu stellen. Weiterhin sollte die Herstellung der Nanoemulsionen einfach und energiegünstig sein. Von Interesse war es insbesondere Nanoemulsionen bereit zu stellen, welche nach der Phaseninversionsmethode (PIT-Verfahren) hergestellt werden können, obwohl sie keine ethoxylierten Emulgatoren enthalten. Eine weitere Aufgabe bestand darin, Nanoemulsionen bereit zu stellen, die einen hohen Anteil an innere Phase aufweisen, im Falle von O/W Emulsionen also einen hohen Anteil an Ölkörpern. Weiterhin war es wünschenswert Nanoemulsionen mit einem möglichst geringen Anteil an Emulgatoren bereitzustellen. Weiterhin sollten die Nanoemulsionen mit einem breiten Spektrum von Ölkörpern herstellbar sein.

Es wurde gefunden, dass Nanoemulsionen gemäß Anspruch 1 diese Aufgaben lösen.

Lipophile Stoffe, wie beispielsweise Vitamine, Parfümöle oder UV-Lichtschutzfilter lassen sich vielfach nur schwer in kosmetische oder pharmazeutische Zubereitungen einarbeiten, insbesondere dann, wenn diese Zubereitungen einen überwiegend polaren Charakter aufweisen. In solchen Fällen kommen Lösungsvermittler zum Einsatz, bei denen es sich um einzelne Stoffe oder Mischungen mit mittleren HLB-Werten handelt, die also gewissermaßen eine Brücke von der polaren Umgebung zum unpolaren Substrat bilden. Die Begriffe Lösungsvermittler, Lösevermittler und Solubilisator werden synonym verwendet. Sehr effektive Lösungsvermittler stellen die Sulfonate kurzkettiger Alkylaromaten, wie z.B. Toluol- oder Cumolsulfonat dar, die wegen ihrer unzureichenden hautkosmetischen Verträglichkeit aber im Bereich der Kosmetik und Pharmazie keine Bedeutung haben. Andere kosmetische Solubilisatoren, wie z.B. spezielle hydrophilisierte Öle, sind zwar hautverträglich, besitzen aber kein ausreichendes Lösungsvermögen und/oder ein schlechtes Kälteverhalten, d.h. zeigen schon bei Raumtemperatur die Tendenz zur Austrübung. Diese hydrophilisierten Öle sind beispielsweise unter der INCI Bezeichnung "Ethoxlyated Hydrogenated Castor Oil" kommerziell erhältlich. Aus diesem Grunde besteht vor allem in der kosmetischen Industrie der Wunsch nach neuen Lösungsvermittlern (Solubilisatoren), die frei von den oben geschilderten Nachteilen sind.

Eine weitere Aufgabe der vorliegenden Erfindung hat somit darin bestanden, neue Lösungsvermittler (Solubilisatoren) zur Verfügung zu stellen, die gegenüber den Produkten des Stands der Technik ein verbessertes Lösungsvermögen, insbesondere gegenüber lipophilen Stoffen, wie z.B. Parfümölen, Vitaminen, UV-Lichtschutzfilter, lipophilen pharmazeutischen Wirkstoffen und dergleichen aufweisen. Von besonderem Interesse war hierbei, dass die Produkte bei Raumtemperatur flüssig sind. Wünschenswert war weiterhin, dass die Produkte keine Polyglykolether enthalten.

### Gegenstand der Erfindung

Ein Gegenstand der Erfindung sind Nanoemulsionen, enthaltend eine Wasserphase und eine Ölphase, dadurch gekennzeichnet, dass sie mindestens einen Alkyl- und/oder Alkenylether von Alkyl- und/oder Alkenyl(poly)glycosiden der Formel (I-A)

(Gₘ-R¹)R²ₙ (I-A)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,0 bis 3,0, vorzugsweise 1,2 bis 1,8, darstellt, und
- n eine Zahl von 0,5 bis 5,0, vorzugsweise 1,4 bis 2,6, darstellt
enthalten.

Ein weiterer Gegenstand der Erfindung sind Alkyl- und/oder Alkenylethergemische von Alkyl- und/oder Alkenylpolyglycosiden der Formel (I-C)

(Gₘ-R¹)R²ₙ (I-C)

- in der G für einen Zuckerrest mit 5 bis 6 C Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten.

Alkylethergemische von Alkyl- und/oder Alkenylpolyglycosiden sind im Stand der Technik beschrieben. Jedoch werden Verbindungen gemäß Forml (I-C) nicht offenbart, ebenso wenig wie ihre Eignung als Solubilisatoren.

EP 0 364 852 A2 **(BASF)** beschreibt substituierte Glucoside der Formel (Gluₘ-R¹)Rₙ², wobei Glu eine Glucose Einheit bezeichnet, R¹ einen C8 bis C18 Alkylrest in Acetalbindung, R² für C1 bis C4 Alkylgruppen mit Etherbindung steht. m hat einen mittleren Wert von 1 bis 10 und n einen mittleren Wert von 0,1m bis 2m. Die in EP 0 365 285 A2 offenbarten Verbindungen tragen maximal 2 Mol Alkylgruppen pro Glucoseeinheit, da n maximal 2m darstellt. Als Edukte werden Glucoside mit einer Wert m von 1 bis 10, vorzugsweise von 1 bis 5 angegeben, insbesondere 1,5 bis 3 (EP 0 364 852 A2, S. 2, Z. 36 sowie Z. 53.) Die Beispiele der EP 0 364 852 A2 haben alle einen mittleren Wert von m von 2,6 bis 2,8. Die in EP 0 364 852 A2 beschriebenen Produkte tragen als Rest R¹ Alkylgruppen mit einer C Kettenlänge von 8 bis 18 C-Atomen, die erfindungsgemäßen Beispiele sind Glucoside aus C10-/C12 Alkanol-Destillationsschritten. Im Gegensatz dazu haben mindestens 50 Gew.-% der erfindungsgemäßen Alkylether von Alkyl- und/oder Alkenylpolyglucosiden R¹ Reste, die einen Alkylrest mit größer gleich 12 C Atomen darstellen. Überraschenderweise wurde gefunden, dass durch Auswahl von Alkylether von Alkyl- und/oder Alkenylpolyglucosiden mit dieser C-Kettenlänge gegenüber dem Stand der Technik verbesserte Solubilisatoren erhalten werden.

US2004/0254084 (McCall**)** beschreibt in Anspruch 1 eine Verbindung aus 2 Glucoseeinheiten, welche eine Alkylgruppe mit 11 C-Atomen am C-1 Atom trägt. Von den in diesem Diglucosid frei verfügbaren 7 OH-Gruppen sind gemäß der Formel in Anspruch 1 4 OH Gruppen mit Alkylgruppen der Kettenlänge C1 bis C8 verethert. Die hier beschriebenen Verbindungen haben demnach einen theoretischen Polymerisationsgrad m von 2 und tragen ausschließlich Reste R1 mit einer C-Kettenlänge von 11.

WO93/06115(Henkel**)** beschreibt ein wasserfreies Verfahren zur Herstellung von Alkyl- und/oder Alkenylpolyglykosidethern, bei dem Alkyl- und/oder Alkenylpolyglykoside der Formel R¹-O-[G]ₚ, in der R¹ für einen aliphatischen, linearen oder verzweigten Kohlenwasserstoffrest mit 6 bis 22 C-Atomen und 0,1,2 oder 3 Doppelbindungen steht, und [G] für einen Zuckerrest mit 5 oder 6 Kohlenstoffatomen und p für Zahlen von 1 bis 10, in Gegenwart alkalischer Verbindungen mit Halogenkohlenwasserstoffen umgesetzt werden. Als Halogenkohlenwasserstoffe werden Alk(en)ylhalogenide mit 1 bis 18 Kohlenstoffatomen sowie Benzylhalogenide beschrieben (S. 4). Gemäß WO 93/06115 (S. 5, 2. Absatz) werden Alkyl- und/oder Alkenylglykoside und Halogenkohlenwasserstoffe im Molaren Verhältnis von 1: 0,9 bis 1:10 eingesetzt, optimalerweise im Molaren Verhältnis von 1:1 bis 1:5. Obwohl in WO 93/06115 auf S. 4, 3. Absatz als Alkylierungsmittel auch Alkylhalogenide, wie beispielsweise Methylchlorid beschrieben werden, ermöglicht die Offenbarung der WO 93/06115 nicht, erfindungsgemäße Verbindungen gemäß Formel (I) zu erhalten: Die Beispiele beschreiben ausschließlich Benzylether, welche durch Umsetzung von C12/C14 Kokosalkylglucosid mit Benzylchlorid erhalten wurden. Benzylchlorid liegt bei Raumtemperatur flüssig vor, im Gegensatz dazu sind die Alkylierungsmittel Methylchlorid oder Ethylchlorid bei Raumtemperatur gasförmig. Wenn man nun, wie in WO 93/06115 beschrieben, wasserfrei arbeitet, muss man das Edukt (Alky- und/oder Alkenylpolyglykosid), welches bei Raumtemperatur ebenfalls fest vorliegt, auf ca. 80 °C erhitzen und dann durch Zufügen von NaOH alkalische Bedingungen einstellen. Man erhält eine hochviskose Mischung, aus der es nicht möglich ist, zusammen mit dem gasförmigen Alkylierungsmittel erfindungsgemäße Produkte zu erhalten. D.h. obwohl die Beschreibung der WO 93/06115 als Verfahrensvariante auch eine Reaktion mit Alkylierungsagentien beschreibt, kann der Fachmann diesem Stand der Technik eine nacharbeitbare Lehr nur für die Aralkylierungsreaktion und somit auch nur für die Aralkylierten Alky- und/oder Alkenylpolyglykoside entnehmen.

US 4,663,444 (Egan**)** beschreibt Ether von Zuckern (Monoglucosid), welche an O1 und O6 Position substituiert sind, wobei die Alkylgruppe an 06 Position 12 bis 18 C-Atome trägt.

### Nanoemulsion

Erfindungsgemäß sollen unter dem Begriff Nanoemulsion Emulsionen mit einer Teilchen- bzw. Tröpfchengröße von unter 1000 nm verstanden werden. Üblicherweise liegt die Teilchengröße der erfindungsgemäßen Nanoemulsion im Bereich von 5 bis 500 nm und insbesondere bei 50 bis 200 nm, vorzugsweise 10 bis 100 nm.

### Alkylether von Alkyl- und/oder Alkenylpolyglycosiden sowie deren Gemische

Die erfindungsgemäße Nanoemulsion enthält mindestens einen Alkyl- und/oder Alkenylether von Alkyl- und/oder Alkenyl(poly)glycosiden der Formel (I-A)

(Gₘ-R¹)R²ₙ (I-A)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,0 bis 3,0 vorzugsweise 1,2 bis 1,8 darstellt, und
- n eine Zahl von 0,5 bis 5,0, vorzugsweise 1,4 bis 2,6 darstellt.

In einer bevorzugten Ausführungsform der Erfindung enthält die Nanoemulsion mindestens ein Alkyl- und/oder Alkenylethergemisch von Alkyl- und/oder Alkenyl(poly)glycosiden der Formel (I-B)

(Gₘ-R¹)R²ₙ (I-B)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,0 bis 3,0 darstellt, vorzugsweise 1,2 bis 1,8 und
- n eine Zahl von 0,5 bis 5,0, vorzugsweise 1,4 bis 2,6 darstellt
wobei mindestens 50 Gew.-% der Alkyl- und/oder Alkenylether einen Rest R¹ mit einer C Kette größer gleich 12 enthalten.

Ein bevorzugter Gegenstand der Erfindung betrifft eine Nanoemulsion enthaltend eine Wasserphase und eine Ölphase, dadurch gekennzeichnet, dass sie mindestens ein Alkyl- und/oder Alkenylethergemisch von Alkyl- und/oder Alkenylpolyglycosiden der Formel (I-C)

(Gₘ-R¹)R²ₙ (I-C)

- in der G für einen Zuckerrest mit 5 bis 6 C Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten.

G steht für einen Zuckerrest (= Monosaccharid) mit 5 oder 6 C-Atomen. Geeignet sind Aldosen, Aldulosen, Hexosen sowie Hexulosen. Exemplarisch für Aldosen mit fünf Kohlenstoff-Atomen (=Pentosen) seinen Xylose, Lyxose, Ribose und Arabinose genannt. Exemplarisch für Aldosen mit sechs Kohlenstoff-Atomen (= Hexosen) seien Glucose, Galactose oder Mannose genannt. Exemplarisch für Ketosen mit einer unverzweigten Kette von sechs Kohlenstoff-Atomen (= Hexulosen) seinen Fructose und Sorbose genannt. Unter den Aldosen ist insbesondere die Glucose bevorzugt. Eine bevorzugte Ausführungsform der Erfindung betrifft demnach Alkylether bzw. Alkylethergemische von Alkyl- und/oder Alkenyl(poly)glucosiden.

Die erfindungsgemäßen Verbindungen stellen Gemische von Alkyl- und/oder Alkenylether von Alkyl- und/oder Alkenylpolyglycosiden dar. Die im Gemisch vorhandenen Alkylether unterscheiden sich durch den Polymerisationsgrad der Zuckereinheiten. Die **Zahl** m in der allgemeinen Formel (I) gibt den Polymerisationsgrad, d.h. die Verteilung von Mono- und Oligoglycosiden an. Während m in einer gegebenen Einzelverbindung stets ganzzahlig sein muss, ist der Wert m für eine bestimmtes Alkyl- und/oder Alkenylpolyglycosid (welches ein Gemisch verschiedener Mono- und Oligoglycoside darstellt) eine analytisch ermittelte Größe, die meistens eine gebrochene Zahl darstellt. Der Polymerisationsgrad m der erfindungsgemäßen Alkylether von Alkyl- und/oder Alkenylpolyglycosiden beträgt 1,0 bis 3,0, vorzugsweise 1,1 bis 2,5, insbesondere 1,1 bis 2,0, vorzugsweise 1,2 bis 1,8, vorzugsweise 1,2 bis 1,5, insbesondere 1,2 bis 1,4. Beträgt der Polymerisationgrad m = 1 handelt es sich um einen reinen Monozucker und somit um einen Alkylether eines Alkyl- und /oder Alkenylmonoglycosids. Dies wird mit der Bezeichnung Alkylether von Alkyl- und/oder Alkenyl(poly)glycosiden verdeutlicht, der sowohl das Monoglycosid als auch die Polyglycoside umfasst.

Der Polymerisationsgrad m der als Edukte einzusetzenden Alkyl- und/oder Alkenyl(poly)glycoside sowie der erfindungsgemäßen Alkyl- und/oder Alkenylether der Alkyl- und/oder Alkenyl(poly)glycoside kann analytisch ermittelt werden, wie beispielsweise beschrieben in **"**Alkyl Polyglycosides"; K. Hill, W. v. Rybinski, G.Stoll (Ed.), VCH Weinheim,1997, S.23 -38**.**

Die Zahl n bestimmt den Alkylierungsgrad der Alkyl- und/oder Alkenylether von Alkyl- und/oder Alkenyl(poly)glycosiden Dieser ist definiert als das Verhältnis von Mol Alkoxygruppen im Gemisch zu Mol Alkyl- und/oder Alkenylether der Alkyl- und/oder Alkenyl(poly)glycoside. Der Alkylierungsgrad der erfindungsgemäßen Verbindungen kann bestimmt werden, indem die Alkoxygruppen quantifiziert werden und ins Verhältnis zu den Alkyl- und/oder Alkenylethern der Alkyl- und/oder Alkenyl(poly)glycoside gesetzt werden. Die Bestimmung der Alkoxygruppen kann beispielsweise nach der Methode von Hodges, beschrieben in: **"**Quantitative Organic Microanalysis", A1 Steyermark, 2.Ed, 1961, Academic Press New York/London; S. 422-424**,** erfolgen oder nach der DIN Methode DIN EN 13268. Der Alkylierungsgrad der erfindungsgemäßen Alkyl- und/oder Alkenylether von Alkyl- und/oder Alkenyl(poly)glycosiden beträgt 0,5 bis 5,0, vorzugsweise 0,8 bis 4,0, insbesondere 1,0 bis 3,0, vorzugsweise 1,4 bis 2,6, insbesondere 1,5 bis 2,5, vorzugsweise 1,5 bis 2,0, insbesondere 1,5 bis 1,9.

Ein weitere Kenngröße, um den Alkylierungsgrad zu beschreiben ist die OH-Zahl (Hydroxylzahl), welche angibt wieviel Milligramm Kaliumhydroxid der Essigsäure-Menge äquivalent sind, die von 1 g Substanz bei der Acetylierung gebunden wird und somit ein Maß für die freien OH-Gruppen darstellt. Die OH-Zahl kann nach der DFG Methode DGF C-V 17a bestimmt werden kann.

Der Rest R¹ steht für einen C6 bis C22, vorzugsweise C8 bis C18 Alkyl- und/oder Alkenylrest in Acetalbindung, wobei R¹ gegebenenfalls hydroxysubstituiert sein kann, wobei R¹ lineare oder verzweigt sein kann. Der Rest R² steht für eine C1 bis C4 Alkyl- und/oder Alkenylgruppe in Etherbindung stehen, die gegebenenfalls verzweigt sein kann. Steht der Rest R² für eine C1 bis C4 Alkylgruppe erhält man Alkylether von Alkyl- und/oder Alkenyl(poly)glykosiden. Der Rest R² kann ebenfalls für einen C1 bis C4 Rest stehen, der ungesättigt ist. Dann enthält man Alkenylether von Alkyl- und/oder Alkenyl(poly)glykosiden. Besonders bevorzugt sind Verbindungen der Formel (I), in denen R² für einen C1 bis C4 Alkylrest steht

In einer bevorzugten Ausführungsform der Erfindung enthält die Nanoemulsion Alkyl- und/oder Alkenylethergemische der allgemeinen Formel (I-B) oder (I-C), in denen mindestens 50 Gew.-% der Alkyl- und/oder Alkenylether einen Rest R¹ mit einer C Kette größer gleich 12 enthalten. In einer bevorzugten Ausführungsform der Erfindung tragen mehr als 50 Gew.-% der Ether (= Alkyl- und/oder Alkenylether von Alkyl- und/oder Alkenyl(poly)glykosiden) einen Rest R¹ mit einer C Kette größer gleich 12, vorzugsweise mehr als 60 Gew.-%, insbesondere mehr als 70 Gew.-%, vorzugsweise mehr als 75 Gew.%. Die Gew.-% beziehen sich auf die Gesamtmenge des Alkyl- und/oder Alkenylethergemischs.

In einer Ausführungsform der Erfindung enthalten die erfindungsgemäßen Ethergemische Ether mit R¹ = C12 (= C12 Rest) und R¹ = C14 (= C14 Rest), und die Summe der Alkyl- und/oder Alkenylether mit R¹ = C12 und C14 macht mehr als 50 Gew.-%, insbesondere mehr als 60 Gew.-%, vorzugsweise mehr als 70 Gew.-%, insbesondere mehr als 75 Gew.-%, vorzugsweise mehr als 80 Gew.-%, bezogen auf die Gesamtmenge des Alkyl- und/oder Alkenylethergemischs aus.

Der Begriff "C12 Rest" umfasst Alkyl- und/oder Alkenylreste mit einer Anzahl von Kohlenstoffatomen von 12. Analog umfasst der Begriff "C14 Rest" Alkyl und/oder Alkenylreste mit einer Anzahl von Kohlenstoffatomen von 14.

Die erfindungsgemäßen Nanoemulsionen enthalten die Alkyl- und/oder Alkenylether bzw. Alkyl- und/oder Alkyenylethergemische von Alkyl- und/oder Alkenylpolyglycosiden üblicherweise in Mengen von 2 bis 25 Gew.-%, insbesondere 3 bis 20 Gew.-%, vorzugsweise 5 bis 10 Gew. % bezogen auf die Nanoemulsion.

### Verfahren zur Herstellung der Alkyl- und/oder Alkenylether von Alkyl- und/oder Alkenyl(poly)glykosiden sowie deren Gemische

Die erfindungsgemäß einzusetzenden Alkyl- und/oder Alkenylether von Alkyl- und/oder Alkenyl(poly)glykosiden können durch Umsetzung von Alkyl- und/oder Alkenyl(poly)glucosiden der Formel (II)

Gₘ-R¹ (II)

- in der G für einen Zuckerrest mit 5 oder 6 C-Atomen steht,
- R¹ für einen C6 bis C22 Alkyl- und/oder Alkenylrest in Acetalbindung steht,
- m einen mittleren Wert von 1 bis 3 darstellt, vorzugsweise 1,2 bis 1,8 und
mit Alkylierungsmittel der Formel (III) R²-X
- in der X eine nucleophile Abhangsgruppe darstellt, und
- R² für eine C1 bis C4 Alkyl- und/oder Alkenylgruppe steht,
   erhalten werden.

Die bevorzugten Alkyl- und/oder Alkylenethergemische von Alkyl- und/oder Alkenyl(poly)glykosiden können durch Umsetzung von Alkyl- und/oder Alkenyl(poly)glucosiden der Formel (II)

Gₘ-R¹ (II)

- in der G für einen Zuckerrest mit 5 oder 6 C-Atomen steht,
- R¹ für einen C6 bis C22 Alkyl- und/oder Alkenylrest in Acetalbindung steht,
- m einen mittleren Wert von 1 bis 3, vorzugsweise 1,2 bis 1,8, darstellt, und wobei mindestens 50 Gew.-% der Alkyl- und/oder Alkenylpolyglycoside einen Rest R¹ mit einer C Kette größer gleich 12 enthalten
mit Alkylierungsmittel der Formel (III) R²-X
- in der X eine nucleophile Abhangsgruppe darstellt, und
- R² für eine C1 bis C4 Alkyl- und/oder Alkenylgruppe steht,
   erhalten werden.

Ein weiterer Gegenstand der Erfindung betrifft ein Verfahren zur Herstellung von Alkyl- und/oder Alkenylethergemischen der allgemeinen Formel (I-C), dadurch gekennzeichnet, dass man Alkyl- und/oder Alkenylpolyglycoside der Formel (II)

Gluₘ-R¹ (II)

- in der G für einen Zuckerrest mit 5 oder 6 C-Atomen steht,
- R¹ für einen C6 bis C22 Alkyl- und/oder Alkenylrest in Acetalbindung steht,
- m einen mittleren Wert von 1,2 bis 1,8 darstellt, und
wobei mindestens 50 Gew.-% der Alkyl- und/oder Alkenylpolyglycoside einen Rest R¹ mit einer C Kette größer gleich 12 enthalten
mit Alkylierungsmittel der Formel (III) R²-X
- in der X eine nucleophile Abhangsgruppe darstellt, und
- R² für eine C1 bis C4 Alkyl- und/oder Alkenylgruppe steht,
umsetzt.

Als Edukte für die erfindungsgemäßen Alkyl- und/oder Alkenylether von Alkyl- und/oder Alkenyl(poly)glykosiden sowie deren Gemische eignen sich Alkyl- und/oder Alkenyloligoglycoside der allgemeinen Formel (II):

Gₘ-R¹ (II)

- in der G für einen Zuckerrest mit 5 oder 6 C Atomen steht,
- R¹ für einen C6 bis C22 Alkyl- und/oder Alkenylrest in Acetalbindung steht,
- m einen mittleren Wert von 1 bis 3, vorzugsweise 1,2 bis 1,8 darstellt.

Als Edukte für die erfindungsgemäßen Alkyl- und/oder Alkenylether von Alkyl- und/oder Alkenyl(poly)glykosiden sowie insbesondere als Edukte für deren Gemische eignen sich Alkyl- und/oder Alkenyloligoglycoside der allgemeinen Formel (II):

Gₘ-R¹ (II)

- in der G für einen Zuckerrest mit 5 oder 6 C Atomen steht,
- R¹ für einen C6 bis C22 Alkyl- und/oder Alkenylrest in Acetalbindung steht,
- m einen mittleren Wert von 1 bis 3, vorzugsweise 1,2 bis 1,8 darstellt, und wobei mindestens 50 Gew.-% der Alkyl- und/oder Alkenylpolyglycoside einen Rest R¹ mit einer C Kette größer gleich 12 enthalten.

Als Edukte für die erfindungsgemäßen Alkyl- und/oder Alkenylether von Alkyl- und/oder Alkenyl(poly)glykosiden sowie insbesondere als Edukte für deren Gemische eignen sich Alkyl- und/oder Alkenyloligoglycoside der allgemeinen Formel (II):

Gₘ-R¹ (II)

- in der G für einen Zuckerrest mit 5 oder 6 C Atomen steht,
- R¹ für einen C6 bis C22 Alkyl- und/oder Alkenylrest in Acetalbindung steht,
- m einen mittleren Wert von 1,2 bis 1,8 darstellt, und
wobei mindestens 50 Gew.-% der Alkyl- und/oder Alkenylpolyglycoside einen Rest R¹ mit einer C Kette größer gleich 12 enthalten.

Diese Edukte können nach den einschlägigen Verfahren der präparativen organischen Chemie erhalten werden. Stellvertretend für das umfangreiche Schrifttum sei hier auf die Schriften EP-A1-0 301 298 und WO 90/03977 verwiesen.

Geeignet als Edukte sind beispielsweise die unter dem Handelsnamen Plantacare®1200 von Fa. Cognis erhältlichen Alkylpolyglucoside (INCI Laurylglucoside).

Der Alkyl- bzw. Alkenylrest R¹ kann sich von primären Alkoholen mit 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Der Alkyl- bzw. Alkenylrest R¹ kann sich ferner auch von primären Alkoholen mit 12 bis 22, vorzugsweise 12 bis 16 Kohlenstoffatomen ableiten. Typische Beispiele sind Laurylalkohol, Isotridecylalkohol, Myristylalkohol, Cetylalkohol, Isocetylalkohol, Palmoleylalkohol, Stearylalkohol, Isostearylalkohol, Ricinolalkohol, Hydroxystearylalkohol, Dihydroxystearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Ebenfalls eingesetzt werden können Guerbetalkohole mit 12 bis 36 Kohlenstoffatomen sowie technische Dimerdiol- und Trimertriol-Gemische mit 18 bis 36 bzw. 18 bis 54 Kohlenstoffatomen.

### Alkylierungs- bzw. Alkenylierungsmittel

Als Alkylierungs- bzw. Alkenylierungsmittel können alle Verbindungen eingesetzt werden, die eine Veretherung der freien OH-Gruppen der Alkyl- und/oder Alkenylpolyglycoside ermöglichen. Beispielsweise seien Verbindungen vom Typ R²-X genannt, wobei X eine nucleophile Abgangsgruppe darstellt. R² stellt die Alkyl- und/oder Alkenylgruppe dar, die über die Sauerstoffgruppe des Alkyl- und/oder Alkenylpolyglycosids in Etherbindung verknüpft ist. R² ist eine C1 bis C4 Alkyl- und/oder Alkenylgruppe. Diese kann linear oder verzweigt sein, exemplarisch seinen genannt Methyl-, Ethyl-, Propyl-, Isopropyl-, n- Butyl-, 2-Methylpropyl (= Isobutyl), 1-Methylpropyl- (= sec-butly) und 1,1-Dimethylethyl- (= tert.-Butyl), Ethenyl- (=Vinyl-), Propen-1-enyl-; Propen-2-enyl-, Isopropenyl-.

Beispiele für geeignete Alkylierungs- bzw. Alkenylierungsmittel vom Typ R²-X sind Alkyl- bzw. Alkenyhalogenide und/oder Alkyl- bzw. Alkenyltosylate und/oder Dialkyl- bzw. Dialkenylsulfate.

Als Alkyltosylate (= Alkyl-p-Toluolsulfonate) seinen exemplarisch genannt Methyltosylat, Ethyltosylat und Benzyltosylat. Als Alkysulfate seinen exemplarisch genannt Dimethylsulfat, Diethylsulfat, Dipropylsulfat und Dibutylsulfat.

Besonders bevorzugt als Alkylierungsmittel sind Alkylhalogenide, insbesondere Chloride und/oder Iodide. In einer bevorzugten Ausführungsform der Erfindung werden die Alkylierungsmittel ausgewählt aus der Gruppe bestehend aus Methylchlorid, Ethylchlorid, n-Propylchlorid, iso-Propylchlorid, n-Butylchlorid, iso-Butylchlorid, sec-Butylchlorid, und tert-Butylchlorid sowie den entsprechenden Brom- und Jodverbindungen.

Es können auch Gemische der Alkylierungs- bzw. Alkenylierungsmittel eingesetzt werden.

### Alkylierungs- bzw. Alkenylierungsreaktion

Die erfindungsgemäßen Alkyl- und/oder Alkenylether von Alkyl- und/oder Alkenyl(poly)glykosiden sowie deren Gemische können durch Umsetzung von Alkyl- und/oder Alkenylpolyglycosiden mit Alkylierungs- bzw. Alkenylierungsmitteln erhalten werden.

Die Alkylierungs- bzw. Alkenylierungsreaktion wird üblicherweise bei 20 bis 100 °C, insbesondere bei 40 bis 90 °C, vorzugsweise bei 60 bis 90 °C durchgeführt. Üblicherweise wird die Reaktion bei erhöhtem Druck, d.h. bei 2 bis 10 bar, vorzugsweise bei 3 bis 5 bar durchgeführt. Als Lösungsmittel eignen sich Wasser oder Gemische aus Wasser mit niedermolekularen Alkoholen, wie beispielsweise Isopropanol oder 1,2 Propylenglykol.

Die Reaktion wird üblicherweise in Gegenwart alkalischer Verbindungen, beispielsweise Alkalimetallhydroxide, wie Natriumhydroxid oder Kalimhydroxid erhalten. Es hat sich als vorteilhaft erwiesen das Alkalimetallhydroxide equimolar oder im molaren Überschuß bezogen auf das Alkylierungs- bzw. Alkenylierungsmittel einzusetzen.

Die erfindungsgemäßen Produkte können erhalten werden, in dem man Alkyl- und/oder Alkenylpolyglycoside mit den Alkylierungs- bzw. Alkenylierungsmitteln beispielsweise im molaren Verhältnis von 1:3 von 1:10, insbesondere von 1:3 bis 1:8 umsetzt.

In einer bevorzugten Ausführungsform der Erfindung werden die Ether bzw. Ethergemische nach der Umsetzung entsalzt. Die Entsalzung kann beispielsweise durch Gefriertrocknung mit nachgeschalteter, gegebenenfalls mehrfacher Extraktion mit Ethanol, durchgeführt werden. Die Entsalzung kann auch mit üblichen Membranverfahren, wie beispielsweise Ultrafiltration oder Diafiltration durchgeführt werden.

Ein weiterer Gegenstand der Erfindung betrifft ein Verfahren zur Herstellung von Alkyl- und/oder Alkenylethergemischen der allgemeinen Formel (I-C), dadurch gekennzeichnet, dass man Alkyl- und/oder Alkenylpolyglycoside der Formel (II)

Gluₘ-R¹ (II)

- in der G für einen Zuckerrest mit 5 oder 6 C-Atomen steht,
- R¹ für einen C6 bis C22 Alkyl- und/oder Alkenylrest in Acetalbindung steht,
- m einen mittleren Wert von 1,2 bis 1,8 darstellt, und
wobei mindestens 50 Gew.-% der Alkyl- und/oder Alkenylpolyglycoside einen Rest R¹ mit einer C Kette größer gleich 12 enthalten
mit Alkylierungsmittel der Formel (III) R²-X
- in der X eine nucleophile Abhangsgruppe darstellt, und
- R² für eine C1 bis C4 Alkyl- und/oder Alkenylgruppe steht,
umsetzt und das so erhaltene Alkyl- und/oder Alkenylethergemisch anschliessend entsalzt.

### Herstellung von Nanoemulsionen

Zur Herstellung von Nanoemulsionen stehen dem Fachmann verschiedene Methoden zu Verfügung. Neben dem Verdünnen von Mikroemulsionen und der Hochdruckhomogenisierung ist die Phaseninversionstemperatur Methode (sog. PIT Methode) die am häufigsten verwendete Methode. Allerdings war die PIT-Methode bisher auf Systeme beschränkt, die zwingend einen ethoxylierten Emulgator enthalten.

Überraschenderweise wurde gefunden, dass mit den Alkyl- und/oder Alkenylether von Alkyl- und/oder Alkenyl(poly)glycosiden der Formel (I-A) und/oder (I-B) stabile Nanoemulsionen erhalten werden.

Ein weiterer Gegenstand der Erfindung betrifft daher die Verwendung von Alkyl- und/oder Alkenylether von Alkyl- und/oder Alkenyl(poly)glycosiden der Formel (I-A)

(Gₘ-R¹)R²ₙ (I-A)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,0 bis 3,0, vorzugsweise 1,2 bis 1,8 darstellt, und
- n eine Zahl von 0,5 bis 5,0, vorzugsweise 1,4 bis 2,6 darstellt
und/oder von Alkyl- und/oder Alkenylethergemischen von Alkyl- und/oder Alkenyl(poly)glycosiden der Formel (I-B)

(Gₘ-R¹)R²ₙ (I-B)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,0 bis 3,0 vorzugsweise 1,2 bis 1,8 darstellt, und
- n eine Zahl von 0,5 bis 5,0, vorzugsweise 1,4 bis 2,6 darstellt wobei mindestens 50 Gew.-% der Alkyl- und/oder Alkenylether einen Rest R¹ mit einer C Kette größer gleich 12 enthalten,
zur Herstellung von Nanoemulsionen, insbesondere zur Herstellung von Nanoemulsionen nach dem PIT-Verfahren.

Ein weiterer Gegenstand der Erfindung betrifft ein Verfahren zur Herstellung von Nanoemulsionen, dadurch gekennzeichnet, dass man entweder
- die Ölphase zusammen mit mindestens einem Alkyl- und/oder Alkenylether von Alkyl- und/oder Alkenyl(poly)glycosiden der Formel (I-A) und/oder (I-B) und/oder (I-C) und der Wasserphase auf eine Temperatur oberhalb der Phaseninversionstemperatur erhitzt und diese Mischung abkühlt oder
- die Ölphase zusammen mit mindestens einem Alkyl- und/oder Alkenylether von Alkyl- und/oder Alkenyl(poly)glycosiden der Formel (I-A) und/oder (I-B) und/oder (I-C) auf eine Temperatur oberhalb der Phaseninversionstemperatur erhitzt und diese durch Zugabe der nicht erhitzten Wasserphase abkühlt.

Die Phaseninversionstemperatur für das erfindungsgemäße Verfahren liegt üblicherweise im Bereich von 15 bis 90 °C. Im Phaseninversionstemperaturbereich kommt es zu einem starken Sprung in der Leitfähigkeit der Mischung. Daher kann die Phaseninversionstemperatur kann für ein gegebenes System einfach durch Leitfähigkeitsmessung ermittelt werden, wie beispielsweise bei P. Izquierdo et al. (Journal of colloid and Interface Science 285, 2005, S. 388-394**)** auf S. 389 unter Punkt 2.2 beschrieben.

Erfindungsgemäß bevorzugt ist eine Abkühlung mit einer Kühlrate von größer 0,2 °C pro Minute, vorzugsweise größer 0,5 °C pro Minute, insbesondere größer 0,7 °C pro Minute, vorzugsweise größer 1 °C pro Minute.

Die lipophile Phase kann neben der Ölphase und den Alkyl- und/oder Alkenylether von Alkyl- und/oder Alkenyl(poly)glycosiden der Formel (I) weitere öllösliche Verbindungen, wie beispielsweise die Co-Emulgatoren, UV-Lichtschutzfilter, Parfümöle etc. enthalten.

Die Wasserphase kann neben Wasser wasserlösliche Verbindungen, wie beispielsweise wasserlösliche Vitamine oder wasserlösliche Wirkstoffe enthalten.

### Verwendung der Nanoemulsionen

Die erfindungsgemäßen Nanoemulsionen eignen als kosmetische und/oder pharmazeutische Zubereitungen oder als Grundlagen für die Herstellung von kosmetischen und/oder pharmazeutischen Zubereitungen. Diese Zubereitungen können in Form von wässrigen Lösungen, Ölen, Emulsionen (W/O oder O/W), Cremes, Lotionen, etc. vorliegen.

### Alkyl- und/oder Alkenylethergemische von Alkyl- und/oder Alkenylpolyglycosiden der Formel (I-C)

Ein weiterer Gegenstand der Erfindung sind Alkyl- und/oder Alkenylethergemische von Alkyl- und/oder Alkenylpolyglycosiden der Formel (I-C)

(Gₘ-R¹)R²ₙ (I-C)

- in der G für einen Zuckerrest mit 5 bis 6 C Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten.

Die erfindungsgemäßen Alkyl- und/oder Alkenylethergemische der Formel (I-C) sind dadurch gekennzeichnet, dass mindestens 50 Gew.-% der Alkyl- und/oder Alkenylether einen Rest R¹ mit einer C Kette größer gleich 12 enthalten. In einer bevorzugten Ausführungsform der Erfindung tragen mehr als 50 Gew.-% der Ether (= Alkyl- und/oder Alkenylether von Alkyl- und/oder Alkenylpolyglykosiden) einen Rest R¹ mit einer C Kette größer gleich 12, vorzugsweise mehr als 60 Gew.-%, insbesondere mehr als 70 Gew.-%, vorzugsweise mehr als 75 Gew.%. Die Gew.-% beziehen sich auf die Gesamtmenge des Alkyl- und/oder Alkenylethergemischs.

In einer Ausführungsform der Erfindung enthalten die erfindungsgemäßen Ethergemische der Formel (I-C) Ether mit R¹ = C12 (= C12 Rest) und R¹ = C14 (= C14 Rest), und die Summe der Alkyl- und/oder Alkenylether mit R¹ = C12 und C14 macht mehr als 50 Gew.-%, insbesondere mehr als 60 Gew.%, vorzugsweise mehr als 70 Gew.%, insbesondere mehr als 75 Gew.%, vorzugsweise mehr als 80 Gew.%, bezogen auf die Gesamtmenge des Alkyl- und/oder Alkenylethergemischs aus.

Der Begriff "C12 Rest" umfasst Alkyl- und/oder Alkenylreste mit einer Anzahl von Kohlenstoffatomen von 12. Analog umfasst der Begriff "C14 Rest" Alkyl und/oder Alkenylreste mit einer Anzahl von Kohlenstoffatomen von 14.

In einer bevorzugten Ausführungsform der Erfindung enthalten die erfindungsgemäßen Alkyl- und/oder Alkenylethergemische der Formel (I-C) weniger als 15 Gew.-%, insbesondere weniger als 10 Gew.%, vorzugsweise weniger als 5 Gew.%, besonders bevorzugt weniger als 2 Gew.-% Salz, insbesondere NaCl. Die Gew.-% beziehen sich auf die Summe der erfindungsgemäßen Alkyl- und/oder Alkenylethergemische der Formel (I-C).

Die erfindungsgemäßen Ethergemische der Formel (I-C) eignen sich für oder zur Herstellung von kosmetischen und/oder pharmazeutischen Zubereitungen. Dabei werden sie beispielsweise als Emulgatoren, vorzugsweise als Solubilisatoren eingesetzt

Die erfindungsgemäßen Ethergemische der Formel (I-C) eignen sich insbesondere für oder zur Herstellung von kosmetischen und/oder pharmazeutischen Zubereitungen in Form von Nanoemulsionen.

Die erfindungsgemäßen Ethergemische der Formel (I-C) eignen sich insbesondere als Solubilisatoren. Sie sind insbesondere geeignet lipophile Stoffe, wie beispielsweise lipophile kosmetische oder lipophile pharmazeutische Wirkstoffe zu solubilisieren.

Ein weiterer Gegenstand betrifft Zubereitungen, enthaltend 0,1 bis 20 Gew.-% eines Ethergemischs der Formel (I-C). Die Gew.-% sind bezogen auf das Gesamtgewicht der Zubereitung. Diese Zubereitungen können beispielsweise kosmetische Zubereitungen sein oder Grundlagen für die Herstellung von pharmazeutischen Zubereitungen. Diese Zubereitungen können in Form von wässrigen Lösungen, Ölen, Emulsionen (W/O oder O/W), Cremes, Lotionen, etc. vorliegen.

### Zubereitungen und Nanoemulsionen

Die erfindungsgemäßen Zubereitungen sowie die erfindungsgemäßen Nanoemulsionen, eignen sich insbesondere auch für leichte, sprühbare Anwendungen und/oder als Bestandteile von Pflegeemulsionen für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung. Sie lassen sich auf Tissues, Papiere, Wipes, Vlies-Produkte, Schwämme, Puffs, Pflaster und Bandagen applizieren, die ihren Einsatz im Bereich der Reinigung, Hygiene und/oder Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes, Toilettenpapier, Erfrischungstücher, After-shave Tücher). Sie lassen sich u.a. auch in Zubereitungen zur Haarpflege, Haarreinigung oder Haarfärbung einsetzen. Sie eignen sich insbesondere als Bestandteile von Zubereitungen der dekorativen Kosmetik, wie beispielsweise Lippenstifte, Augen-Make up, wie beispielsweise Lidschatten, Mascara, Lidstifte, Kajal, Nagellack, etc. sowie Make-up Formulierungen.

### Co-Emulgatoren

In einer bevorzugten Ausführungsform der Erfindung enthalten die Nanoemulsionen mindestens einen weiteren Emulgator (=Co-Emulgator).

Ein weiterer Gegenstand der Erfindung betrifft Zubereitungen enthaltend Alkyl- und/oder Alkenylethergemisch von Alkyl- und/oder Alkenylpolyglycosiden der Formel (I-C)

(Gₘ-R¹)R²ₙ (I-C)

- in der G für einen Zuckerrest mit 5 oder 6 C-Atomen steht,
- R¹ für einen C6 bis C22 Alkyl- und/oder Alkenylrest in Acetalbindung steht,
- R² für eine C 1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten.
und mindestens einen Emulgator (=Co-Emulgator).

Die erfindungsgemäßen Nanoemulsionen bzw. die erfindungsgemäßen Zubereitungen enthalten den/die Co-Emulgatoren üblicherweise in einer Menge von 0,01 bis 40 Gew.-%., vorzugsweise 0,05 bis 30 Gew.-% insbesondere 0,05 bis 20 Gew.%, vorzugsweise 0,1 bis 15 Gew.-% und insbesondere 0,1 bis 10 Gew.-% , insbesondere 0,5 bis 5 Gew.-% bezogen die Nanoemulsion bzw. auf die Zubereitung.

In einer bevorzugten Ausführungsform der Erfindung beträgt das Gewichtsverhältnis von Co-Emulgatoren zu den Alkyl- und/oder Alkenylethergemisch von Alkyl- und/oder Alkenyl(poly)glycosiden 0 bis 1,0, vorzugsweise 0 bis 0,7, insbesondere 0 bis 0,5.

Als Co-Emulgator eignet sich prinzipiell jede oberflächenaktive Substanz, insbesondere jedoch Substanzen mit einem HLB-Wert von 1 bis 20 nach der Griffin Skala. Jedem Emulgator wird ein so genannter HLB-Wert (eine dimensionslose Zahl zwischen 1 und 20, Griffin Skala) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Größe und Stärke der hydrophilen und der lipophilen Gruppen eines Emulgators aus. Die Griffin Skala ist beschrieben in WC Griffin, J. Soc. Cosmet. Chem. 1 (1949) 311**;** WC Griffin, J. Soc. Cosmet. Chem. 5 (1954) 249**.**

Der HLB-Wert eines Emulgators lässt sich auch aus Inkrementen errechnen, wobei die HLB-Inkremente für die verschiedenen hydrophilen und hydrophoben Gruppen, aus denen sich ein Molekül zusammensetzt. In der Regel kann er Tabellenwerken (z. B. H. P. Fiedler, Lexikon der Hilfsstoffe für Pharmazie, Kosmetik und angrenzende Gebiete, Editio Cantor Verlag, Aulendorf, 4. Aufl. 1996) oder den Herstellerangaben entnommen werden. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Ölphase entsteht unter sonst gleichen Herstellungsbedingungen eine W/O-Emulsion.

In einer Ausführungsform der Erfindung enthält die erfindungsgemäße Zubereitung mehr als einen Co-Emulgator. Der Fachmann setzt in Abhängigkeit der übrigen Komponenten übliche Emulgator Systeme (wie z.B. Emulgator und Co-Emulgator) ein.

Erfindungsgemäß bevorzugt als als Co-Emulgatoren sind Verbindungen mit einem HLB-Wert von kleiner gleich 11, vorzugsweise kleiner gleich 10. Als Co-Emulgatoren eignen sich insbesondere Verbindungen mit einem HLB-Wert von 1 bis 9.

In einer bevorzugten Ausführungsform der Erfindung enthalten die Nanoemulsionen bzw. die Zubereitungen weniger als 10 Gew.%, vorzugsweise weniger als 5 Gew.%, insbesondere weniger als 2 Gew.-%, insbesondere weniger als 0,5 Gew.-% ethoxylierte Emulgatoren enthält.

Aus diesem Grund sind von denen im Folgenden genannten nicht-ionischen Emulgatoren insbesondere die nicht-ethoxylierten Vertreter der Gruppen (3) und (4), sowie die Vertreter der Gruppe (6) sowie (8), (9) und (10) geeignet.

### Nicht-ionische Emulgatoren

Zur Gruppe der nicht-ionischen Emulgatoren gehören beispielsweise:
(1) Anlagerungsprodukte von 0 bis 50, insbesondere von 2 bis 50 Mol Ethylenoxid und/oder 0 bis 20, insbesondere von 1 bis 20 Mol Propylenoxid an lineare und/oder verzweigte Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls^{®} PGPH" (W/O-Emulgator) oder "Eumulgin^{®} VL 75" (Abmischung mit Lauryl Glucosides im Gewichtsverhältnis 1:1, O/W-Emulgator) oder Dehymuls^{®} SBL (W/O-Emulgator) von der Cognis Deutschland GmbH vertrieben werden. In diesem Zusammenhang sei insbesondere auf das Europäische Patent EP766 661B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Besonders bevorzugte Emulgatoren sind z. B. Cetyl Dimethicone Copolyol (z.B. Abil EM-90), Polyglyceryl-2 Dipolyhydroxystearate (z.B. Dehymuls PGPH), Polyglycerin-3-Diisostearate (z.B. Lameform TGI), Polyglyceryl-4 Isostearate (z.B. Isolan GI 34), Polyglyceryl-3 Oleate (z.B. Isolan GO 33), Glyceryl Stearate Citrate (z. B. Axol C 62, Imwitor 370 bzw. 372P,Dracorin CE 614035), Diisostearoyl Polyglyceryl-3 Diisostearate (z.B. Isolan PDI), Polyglyceryl-3 Methylglucose Distearate (z.B. Tego Care 450), Polyglyceryl-3 Beeswax (z.B. Cera Bellina), Polyglyceryl-4 Caprate (z.B. Polyglycerol Caprate T2010/90), Polyglyceryl-3 Cetyl Ether (z.B. Chimexane NL), Polyglyceryl-3 Distearate (z. B. Cremophor GS 32) und Polyglyceryl Polyricinoleate (z.B. Admul WOL 1403), Glyceryl Oleate (z.B. Monomuls 90-O 18), Alkyl Glucoside (z.B. Plantacare 1200, Emulgade PL 68/50, Montanov 68, Tego Care CG 90, Tego Glucosid L 55), Methyl Glucose Isostearate (z.B. Tego Care IS), Methyl Glucose Sesquistearate (Tego Care PS), Sodium Cocoyl Hydrolyzed Wheat Protein (z.B. Gluadin WK), Potassium Cetyl Phosphate (z.B. Amphisol K, Crodafos CKP), Sodium Alkylsulfate (z.B. Lanette E), Sucrose Ester (z.B. Crodesta F-10, F-20, F-50, F-70, F-110, F-160, SL-40, Emulgade® Sucro), ethoxylierte und/oder propoxylierte Fettalkohole Fettsäuren, Rizinusöle bzw. hydrierte Rizinusöle (z.B. Eumulgin B2, B2, B3, L, HRE 40, HRE 60, RO 40, Cremophor HRE 40, HRE 60, L, WO 7, Dehymuls HRE 7, Arlacel 989), PEG-30 Dipolyhydroxystearate (z.B. Arlacel P 135, Dehymuls LE), Sorbitan Ester, Sorbitan Ester ethoxyliert und/oder propoxyliert sowie deren Gemische. Ein besonders effektives Gemisch besteht aus Polyglyceryl-2 Dipolyhydroxystearate und Lauryl Glucoside und Glycerin (z.B. Eumulgin VL 75). Geeignet sind weiterhin Polyglyceryl-4 Diisostearate/Polyhydroxystearate/Sebacate (Isolan^{®} GPS), Diisostearoyl Polyglyceryl-3 Diisostearate (z. B. Isolan PDI), Alkalisalze Acylglutamate (z.B. Eumulgin SG).

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer,"Encyclopedia of Chemical Technology", 3. Aufl., 1979, Band 8, Seite 913**,** aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8-18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und - oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur: 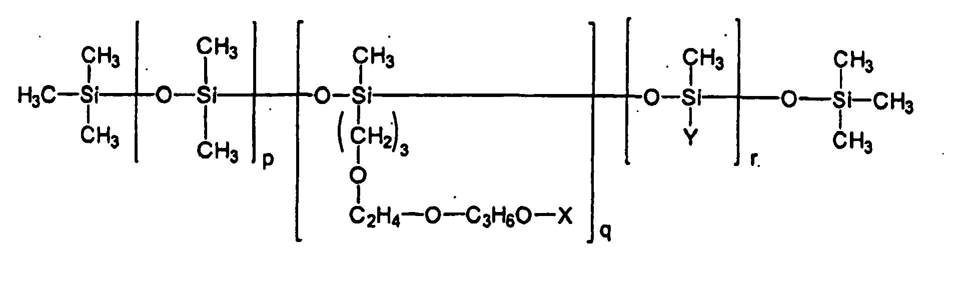 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden.

Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikGoldschmidt unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopo- lyol, welches von Evonik Goldschmidt unter der Warenbezeichnung ABIL®EM 97 und ABIL®WE 09 verkauft wird.
Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Coming Ltd. erhältlich ist.

Ein weiterer vorteilhafter Silikonemulgator ist, Octyl Dimethicon Ethoxy Glucosid der Firma Wacker. Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z.B. ABIL® EM 90 Evonik Goldschmidt), DC5200 Formulation Aid (Dow Coming)] sowie beliebige Mischungen aus beiden Emulgatoren.

Als Co-Emulgator können ebenfalls Tenside eingesetzt werden. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Als Co-Emulgator können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine -COO⁽⁻⁾- oder-SO₃⁽⁻⁾ -Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

Anionische Tenside sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkyloligoglucosidcarboxylate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretriethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

### Ölphase

Die erfindungsgemäßen Nanoemulsionen enthalten eine Ölphase.

Ein weiterer Gegenstand der Erfindung betrifft Zubereitungen enthaltend Alkyl- und/oder Alkenylethergemisch von Alkyl- und/oder Alkenylpolyglycosiden der Formel (I-C)

(Gₘ-R¹)R²ₙ (I-C)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten.
und mindestens eine Ölphase.

Die Ölphase ist üblicherweise in Mengen von von 0,1 - 90, insbesondere 0,1 - 80, insbesondere 0,5 bis 70, bevorzugt 1 bis 60, insbesondere 1 bis 50 Gew.-%, insbesondere 1 bis 40 Gew.-%, vorzugsweise 5-25 Gew.-% und insbesondere 5-15 Gew.-%, bezogen die Nanoemulsion bzw. bezogen auf die Zubereitung enthalten.

In einer bevorzugten Ausführungsform der Erfindung beträgt das Gewichtsverhältnis der Summe aller Co-Emulgatoren und aller Alkyl- und/oder Alkenylether von Alkyl- und/oder Alkenyl(poly)glycosiden zur Summe der Ölphase 0,01 bis 1,0, vorzugsweise 0,05 bis 0,75, insbesondere 0,07 bis 0,5.

Für die Berechnung der Ölphase werden die Alkyl- und/oder Alkenylethergemisch von Alkyl- und/oder Alkenyl(poly)glycosiden der Formel (I-A), (I-B) und (I-C) sowie die gegebenenfalls vorhandenen weiteren Emulgatoren nicht mitberechnet.

Die Ölphase kann Ölkörper, Fett und Wachse/Wachskomponenten enthalten:

Als Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäwen, flüssige Mono-/Di/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetio10 CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen und Kohlenwasserstoffen oder deren Gemische.

Als Ölkörper kommen ebenfalls in Frage Ester von Ester von linearen C6-C22-Fettsäuren mit linearen oder verzwiegten C6- C22-Fettalkoholen, insbesondere Ester von C8-Fettsäuren mit 2-Propylheptanol, wie sie unter dem Handelsnahmen Cetiol®SenSoft (INCI: Propylheptylcarpylate) der Fa. Cognis erhältlich sind.

Als Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 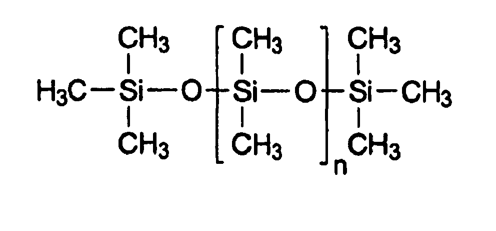 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handelsbezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclotrisiloxan, Polydimethylsiloxan, Poly(methylphenylsiloxan). Erfindungsgemäß besonders bevorzugte Silikone sind Dimethicon und Cyclomethicon.

Unter dem Begriff **Wachs/Wachskomponente** werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 °C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten TriglyceridGemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 18 (C18-Alkohol), Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zusammensetzungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oder -stearat kommen hierfür in Frage.

Als Wachse eignen sich weiterhin Perlglanzwachse. Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Überraschenderweise wurde gefunden, dass sich die erfindungsgemäßen Nanoemulsionen insbesondere eignen, um öllösliche UV-Lichtschutzfilter zu solubilisieren. Ein Gegenstand der Erfindung betrifft Zubereitungen, enthaltend Nanoemulsionen nach Anspruch 1 und mindestens einen UV-Lichtschutzfilter, vorzugsweise einen öllöslichen UV-Lichtschutzfilter.

Überraschenderweise wurde gefunden, dass sich die erfindungsgemäßen Ethergemische der Formel (I-C) insbesondere eignen, um öllösliche UV-Lichtschutzfilter zu solublisieren.

Ein Gegenstand der Erfindung betrifft Zubereitungen, enthaltend Alkyl- und/oder Alkenylethergemisch von Alkyl- und/oder Alkenylpolyglycosiden der Formel (I-C)

(Gₘ-R¹)R²ₙ (I-C)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten, und mindestens einen UV-Lichtschutzfilter, vorzugsweise einen öllöslichen UV-Lichtschutzfilter.

Erfindungsgemäß sind als **UV-Lichtschutzfilter** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
➢ 3-(4'-Trimethylammonium) benzyliden- bornan-2-on-methylsulfat (Mexoryl SO)
➢ 3,3'-(1,4-Phenylendimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)- [2-oxoborn-3-yliden)methyl}benzyl]acrylamid (Mexoryl SW) ➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl XL)
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb® HEB);
➢ 2,2(-Methylen-bis(6-(2H-benzotriazol-2-yl)-4- (1,1,3,3-tetramethyl-butyl)phenol) (Tinosorb M);
➢ 2,4-Bis[4-(2-ethylhexyloxy)-2- hydroxyphenyl]-6-(4- methoxyphenyl)-1,3,5- triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bomyliden)sulfonsäure und deren Salze.

In einer bevorzugten Ausführungsform der Erfindung enthalten die Nanoemulsionen bzw. die erfindungsgemäßen Zubereitungen mindestens einen öllöslichen UV-Lichtschutzfilter sowie mindestens einen wasserlöslichen UV-Lichtschutzfilter.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 **(BASF)** sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der **Fassung Commission Directive 2005/9/EC of 28 January 2005 amending Council Directive 76/768/EEC,** concerning cosmetic products, for the purposes of adapting Annexes VII thereof to technical progress) zugelassen Stoffe, auf die hier explizit Bezug genommen wird.

Die erfindungsgemäßen Nanoemulsionen sowie die erfindungsgemäßen Zubereitungen können auch unlösliche Lichtschutzpigmente, nämlich feindisperse Metalloxide bzw. Salze enthalten. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 8/1996, S. 543-548 **sowie** Parf.Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydro-guajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

In einer bevorzugten Ausführungsform der Erfindung enthalten die Nanoemulsionen bzw. die erfindungsgemäßen Zubereitungen mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden- bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzylidenbornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy)disiloxanyl)propyl)phenol, Dimethicodiethylbenzalmalonate und ihren Mischungen.

Diese UV-Lichtschutzfilter sind beispielsweise unter den folgenden Handelsnamen kommerziell erhältlich:

NeoHeliopan®MBC (INCI: 4-Methylbenzylidene Camphor; Hersteller: Symrise); NeoHeliopan® BB (INCI: Benzophenone-3, Hersteller: Symrise); Parsol®1789 (INCI: Butyl Methoxydibenzoylmethane, Hersteller: Hoffmann-La Roche (Givaudan); Tinosorb®S (INCI: Bis-Ethylhexyloxyphenol Methoxyphenyl Triazine); Tinosorb®M (INCI: Methylene Bis-Benzotriazolyl Tetramethylbutylphenol): Herstelller: Ciba Specialty Chemicals Corporation; Uvasorb®HEB (INCI: Diethylhexyl Butamido Triazone, Hersteller: 3V Inc.), Uvinul®T 150 (INCI: Ethylhexyl Triazone, Hersteller: BASF AG); Uvinul® A plus (INCI: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCI: Camphor Benzalkonium Methosulfate; Mexoryl®SX: 3,3'-(1,4-Phenylendimethin)-bis (7,7- dimethyl-2-oxobicyclo-[2.2.1] heptan-1-methansulfonsäure), CTFA: INCI Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCI Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy) disiloxanyl)propyl) phenol; INCI: DROMETRIZOLE TRISILOXANE; Parsol® SLX: Dimethicodiethylbenzalmalonate, INCI Polysilicone-15.

Die erfindungsgemäßen Nanoemulsionen bzw. die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen die Nanoemulsion bzw. bezogen auf die erfindungsgemäße Zubereitung - enthalten.

Überraschenderweise wurde gefunden, dass sich die erfindungsgemäßen Nanoemulsionen insbesondere eignen um öllösliche Vitamine zu solubilisieren.

In einer bevorzugten Ausführungsform der Erfindung enthalten die Nanoemulsionen mindestens ein Vitamin, vorzugsweise ein öllösliches Vitamin.

Überraschenderweise wurde gefunden, dass sich die erfindungsgemäßen Ethergemische der Formel (I-C) insbesondere eignen, um Vitamine, vorzugsweise öllösliche Vitamine zu solublisieren.

Ein Gegenstand der Erfindung betrifft Zubereitungen, enthaltend Alkyl- und/oder Alkenylethergemisch von Alkyl- und/oder Alkenylpolyglycosiden der Formel (I-C)

(Gₘ-R¹)R²ₙ (I-C)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten, und mindestens ein Vitamin, vorzugsweise ein öllösliches Vitamin

Als geeignete öllösliche Vitamine seien genannt Vitamin A, Vitamin D, Vitamin E und Vitamin K. Weiterhin sei L-Ascorbylpalmitat genannt. Besonders bevorzugt als öllösliches Vitamin ist Vitamin E. Der Begriff Vitamin E ist eine Sammelbezeichnung für α-Tocopherol, β-tocopherol, γ-Tocopherol, δ-Tocopherol und α-Tocotrienol, β-Tocotrienol, γ-Tocotrienol und δ-Tocotrienol. Umfasst sind auch die jeweiligen Tocopherolacetate und Tocoperholpalmitate.

Als geeigneten wasserlösliche Vitamine seien genannt L-Ascorbinsäure (Vitamin C), und die Vitamine der B-Gruppe: Thiamin (Vitamin B₁), Riboflavin (Vitamin B₂, Vitamin G), Niacin (Vitamin B₃), Pantothensäure (Vitamin B₃, Vitamin B₅), Vitamin B₆, Biotin (Vitamin B₇, Vitamin H), Folsäure (Vitamin B₉, Vitamin B_{c} oder Vitamin M) und Vitamin B₁₂.

Überraschenderweise wurde gefunden, dass sich die erfindungsgemäßen Nanoemulsionen insbesondere eignen, um Parfümöle zu solubilisieren.
In einer bevorzugten Ausführungsform der Erfindung enthalten die Nanoemulsionen mindestens ein Parfümöl, vorzugsweise ein öllösliches Parfümöl.

Überraschenderweise wurde gefunden, dass sich die erfindungsgemäßen Ethergemische der Formel (I-C) insbesondere eignen, um Parfümöle, insbesondere öllösliche Parfümöle zu solublisieren.

Ein Gegenstand der Erfindung betrifft Zubereitungen, enthaltend Alkyl- und/oder Alkenylethergemisch von Alkyl- und/oder Alkenylpolyglycosiden der Formel (I-C)

(Gₘ-R¹)R²ₙ (I-C)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten, und mindestens ein Parfümöl, vorzugsweise ein öllösliches Parfümöl.

Unter dem Begriff "Parfümöle" werden einzelne natürlichen oder synthetischen Riechstoffe sowie Gemische aus natürlichen oder synthetischen Riechstoffen, Aromen sowie Gemische von Riechstoffe und Aromen zusammengefasst. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzyl-salicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Je nach Applikationszweck enthalten die Nanoemulsionen bzw. die erfindungsgemäßen Zubereitungen eine Reihe Hilfs- und Zusatzstoffe, wie beispielsweise Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, weitere Solubilisatoren, Konservierungsmittel, Farbstoffe etc..

Ein Gegenstand der Erfindung betrifft Zubereitungen, enthaltend Alkyl- und/oder Alkenylethergemisch von Alkyl- und/oder Alkenylpolyglycosiden der Formel (I-C)

(Gₘ-R¹)R²ₙ (I-C)

- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten, und mindestens eine Verbindung ausgewählt aus der Gruppe bestehend aus Polymeren, Konservierungsmitteln, Antiperspirant/Desodorant Wirkstoffen, Selbstbräunern, Farbstoffen, Stabilisatoren, Hydrotropen, biogenen Wirkstoffen, Insekten-Repellent und/oder Tyrosinaseinhibitoren.

In einer Ausführungsform der Erfindung enthalten die erfindungsgemäßen Nanoemulsionen bzw. die erfindungsgemäßen Zubereitungen mindestens ein **Polymer**. Als Polymere eigenen sich beispielsweise kationische, anionische, zwitterionische, amphotere und nichtionische Polymere.

Geeignete kationische Polymere sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxy-propyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen sowie beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Ebenso geeignet sind sogenannte quaternäre Polymere, z.B. mit der INCI - Bezeichnung Polyquaternium-37, die der folgenden allgemeinen Formel entsprechen: Alternativ können auch andere Dialkylaminoalkyl (meth)acrylate sowie deren durch Alkylierung oder Protonierung erhältliche Ammoniumsalze oder Dialkylaminoalkyl (meth)acrylamide sowie deren durch Alkylierung oder Protonierung erhältliche Ammoniumsalze eingesetzt werden. Besonders bevorzugt sind Polymere enthaltend MAPTAC, APTAC, MADAME, ADAME, DMAEMA und TMAEMAC. Darüberhinaus können auch Co-polymere mit anionischen, weiteren kationischen oder ungeladenen 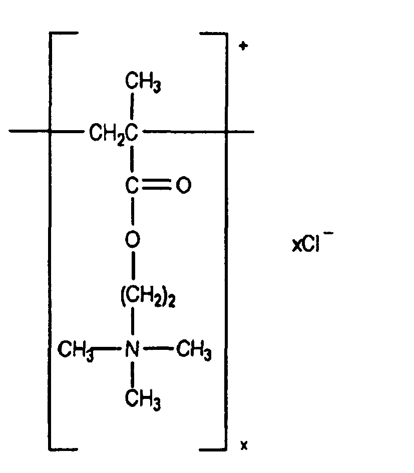 Monomeren erfindungsgemäß eingesetzt werden, insbesondere solche, die neben den genannten Alkylaminoalkyl (meth)acrylat oder -(meth)acryamid Monomeren zusätzlich (Meth)acrylsäure und/oder 2-Acrylamido-2-methyl-propansulfonsäure und/oder Acrylamid und/oder Vinylpyrrolidon und/oder Alkyl(meth)acrylate enthalten.

Beispielhaft seien solche Polymere mit der INCI Bezeichnung Polyquaternium-11, Polyquaternium-13, Polyquaternium-14, Polyquaternium-15, Polyquaternium-28, Polyquaternium-32, Polyquaternium-43, Polyquaternium-47 genannt.

In einer Ausführungsform der Erfindung enthalten die erfindungsgemäßen Nanoemulsionen bzw. die erfindungsgemäßen Zubereitungen mindestens ein **Konservierungsmittel**. Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe. Weiterhin eignen sich als Konservierungsmittel die in WO07/048757 beschriebenen 1,2 Alkandiole mit 5 bis 8 C-Atomen.

Als Konservierungsmittel eignen sich insbesondere die gemäß Annex VI der Kommissions Direktive (in der **Fassung Commission Directive 2007/22/EC of 17 April 2007 amending Council Directive 76/768/EEC**, concerning cosmetic products, for the purposes of adapting Annexes IV and VI thereto to technical progress) zugelassen Stoffe, auf die hier explizit Bezug genommen wird.

In einer Ausführungsform der Erfindung enthalten die erfindungsgemäßen Nanoemulsionen bzw. die erfindungsgemäßen Zubereitungen mindestens einen **Antiperspirant/Desodorant Wirkstoff**.

Erfindungsgemäß sind als Antiperspirant/Desodorant Wirkstoff alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend Antiperspirantien, Esteraseinhibitoren, bakterizide bzw. bakteriostatische Wirkstoffe und/oder schweißabsorbierende Substanzen.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt.

Die erfindungsgemäßen Nanoemulsionen bzw. die erfindungsgemäßen Zubereitungen können die Antiperspirantien in Mengen von 1 bis 50, vorzugsweise 5 bis 30 und insbesondere 8 bis 25 Gew.-% - bezogen auf die Nanoemulsion bzw. bezogen auf die erfindungsgemäße Zubereitung - enthalten.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Nanoemulsionen bzw. die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf die Nanoemulsion bzw. bezogen auf die erfindungsgemäße Zubereitung - enthalten.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid. Die erfindungsgemäßen Nanoemulsionen bzw. die erfindungsgemäßen Zubereitungen können die bakteriziden bzw. bakteriostatischen Wirkstoffe in Mengen von 0,01 bis 5 und vorzugsweise 0,1 bis 2 Gew.-% - bezogen auf die Nanoemulsionen bzw. bezogen auf die erfindungsgemäße Zubereitung - enthalten.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen.

Die erfindungsgemäßen Nanoemulsionen bzw. die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf die Nanoemulsionen bzw. bezogen auf die erfindungsgemäße Zubereitung - enthalten.

In einer Ausführungsform der Erfindung enthalten die erfindungsgemäßen Nanoemulsionen bzw. die erfindungsgemäßen Zubereitungen mindestens einen **Selbstbräuner**.

Als Selbstbräuner sind Substanzen zu verstehen, welche eine Bräunung der Haut verursachen. Beispielsweise seien sowie alpha, beta-ungestättige Aldehyde genannt, welche mit den Aminosäuren der Haut im Sinne einer Maillard Reaktion zu gefärbten Verbindungen abreagieren. Als Wirkstoffe für Selbstbräuner kommen weiterhin in Frage natürliche oder synthetische Ketole oder Aldole. Als geeignete Wirkstoffe seien exemplarisch genannt Dihydroxyaceton, Erythrulose Glycerolaldehyd, Alloxan, Hydroxymethylglyoxal, gamma-Dialdehyd, 6-Aldo-D-Fructose, Ninhydrin und meso-Weinsäuredialdehyd. Als Selbstbräuner eignen sich insbesondere Dihydroxyaceton und/oder Erythrulose.

Als besonders vorteilhaft haben sich Mischungen der o.g. Wirkstoffe untereinander oder mit Mucondialdehyd oder/und Naphthochinone wie z. B. 5-Hydroxy-1,4-naphthochinon (Juglon) und 2-Hydroxy-1,4-Naphthochinon erwiesen. Die erfindungsgemäßen Nanoemulsionen bzw. die erfindungsgemäßen Zubereitungen enthalten die Selbstbräuner üblicherweise in Konzentrationen von 1 bis 10, insbesondere von 2 bis 5 Gew.-% -bezogen auf die Nanoemulsion bzw. bezogen auf die erfindungsgemäße Zubereitung.

In einer Ausführungsform der Erfindung enthalten die Nanoemulsionen bzw. die erfindungsgemäßen Zubereitungen mindestens einen **Farbstoff**.

Die Farbstoffe können sowohl synthetischen als auch natürlichen Ursprungs sein. Eine Liste von geeigneten Farbstoffen findet sich in EP 1 371 359 A2, S.8, Z. 25-57, S.9 und S.10 sowie

S.11, Z. 1 bis 54, auf welche hiermit explizit Bezug genommen wird. Die erfindungsgemäßen Nanoemulsionen bzw. die erfindungsgemäßen Zubereitungen enthalten üblicherweise 0,01 bis 5, vorzugsweise 0,1 bis 1,0 Gew.-% Farbstoffe- bezogen auf die Nanoemulsion bzw. bezogen auf die erfindungsgemäße Zubereitung.

Als Farbstoffe eignen sich insbesondere die gemäß Annex IV der Kommissions Direktive zugelassenen Farbstoffe (in der Fassung: **Commission Directive 2007/22/EC of 17 April 2007 amending Council Directive 76/768/EEC**, concerning cosmetic products, for the purposes of adapting Annexes IV and VI thereto to technical progress) zugelassen Stoffe, auf die hiermit explizit Bezug genommen wird.

In einer Ausführungsform der Erfindung enthalten die erfindungsgemäßen Nanoemulsionen bzw. die erfindungsgemäßen Zubereitungen mindestens einen **Stabilisator**. Als Stabilisatoren können Metallsalze von Fettsäuren, wie z. B. Magnesium-, Aluminium- und/oder Zinkstearat bzw. -ricinoleat eingesetzt werden.

In einer Ausführungsform der Erfindung enthalten die erfindungsgemäßen Nanoemulsionen bzw. die erfindungsgemäßen Zubereitungen mindestens einen **Hydrotrop**. Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

In einer Ausführungsform der Erfindung enthalten die erfindungsgemäßen Nanoemulsionen bzw. die erfindungsgemäßen Zubereitungen mindestens einen **biogenen Wirkstoff**. Unter biogenen Wirkstoffen sind beispielsweise (Desoxy)Ribonucleinsäure und deren Fragmentierungsprodukte, β-Glucane, Bisabolol, Allantoin, Phytantriol, Panthenol, AHA-Säuren, Aminosäuren, Ceramide, Pseudoceramide, essentielle Öle, Pflanzenextrakte, wie z. B. Aloe vera, Prunusextrakt, Bambaranussextrakt zu verstehen.

In einer Ausführungsform der Erfindung enthalten die erfindungsgemäßen Nanoemulsionen bzw. die erfindungsgemäßen Zubereitungen mindestens ein **Insekten-Repellent**. Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

In einer Ausfiihrungsform der Erfindung enthalten die erfindungsgemäßen Nanoemulsionen bzw. die erfindungsgemäßen Zubereitungen mindestens einen **Tyrosinaseinhibitor**. Als Tyrosinasehinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Beispiele

### Herstellbeispiel 1

In einem geeigneten Druckreaktor wurden 290 g (0,36 Mol) Plantacare® 1200, 227 g (2,1 Mol) NaOH (37 %ig) und 30 g Isopropanol vorgelegt und auf 85 °C erhitzt. Anschließend wurden bei einem Druck von maximal 3,3 bar 70 g (1,39 Mol) Methylchlorid portionsweise zudosiert. Nach einer Dosierzeit von 9 Stunden war die Reaktion beendet. Zur Entfernung des bei der Reaktion entstandenen NaCl wurde das Produkt gefriergetrocknet und mehrfach mit Ethanol extrahiert. Die vereinigten Extrakte wurden im Rotationsverdampfer vom Ethanol befreit und in 70 % Wasser aufgenommen.

Das erhaltene klare Produkt hatte einen Polymerisationsgrad m von 1,4; einen Anteil von 90 Gew.-% Alkylether mit einer C-Kette von größer gleich 12 bezogen auf das Alkylethergemisch sowie einen Methylierungsgrad n = 1,7.

### Herstellbeispiel 2

In einem geeigneten Druckreaktor wurden 290 g (0,36 Mol) Plantacare® 1200 und 302 g (2,8 Mol) NaOH (37 %ig) vorgelegt und auf 85 °C erhitzt. Anschließend wurden bei einem Druck von maximal 3,3 bar 110g (2,18 Mol) Methylchlorid portionsweise zudosiert. Nach einer Dosierzeit von 12 Stunden war die Reaktion beendet. Zur Entfernung des bei der Reaktion entstandenen NaCl wurde das Produkt gefriergetrocknet und mehrfach mit Ethanol extrahiert. Die vereinigten Extrakte wurden im Rotationsverdampfer vom Ethanol befreit und in 70 % Wasser aufgenommen. Das erhaltene klare Produkt besaß einen Methylierungsgrad von 2,5.

Das erhaltene klare Produkt hatte einen Polymerisationsgrad m von 1,4; einen Anteil von 90 Gew.-% Alkylether mit einer C-Kette von größer gleich 12 bezogen auf das Alkylethergemisch sowie einen Methylierungsgrad n = 2,5.

### Herstellbeispiel 3

In einem geeigneten Druckreaktor wurden 1700 g (2,08 Mol) Plantacare® 1200, 1700 g Wasser und 832,2 g (10,4 Mol) NaOH (50 %ig) vorgelegt und auf 60 °C erhitzt. Anschließend wurden bei einem Druck von maximal 5 bar 525,3 g (10,4 Mol) Methylchlorid portionsweise zudosiert. Nach einer Dosierzeit von 10 Stunden war die Reaktion beendet. Zur Entfernung des bei der Reaktion entstandenen NaCl wurde das Produkt gefriergetrocknet und mehrfach mit Ethanol extrahiert. Die vereinigten Extrakte wurden im Rotationsverdampfer vom Ethanol befreit und in 70 % Wasser aufgenommen.

Das erhaltene klare Produkt hatte einen Polymerisationsgrad m von 1,4; einen Anteil von 90 Gew.-% Alkylether mit einer C-Kette von größer gleich 12 bezogen auf das Alkylethergemisch sowie einen Methylierungsgrad n = 1,7.

Das in den Beispielen eingesetzte Plantacare®1200 (Fa. Cognis) ist ein Alkylpolyglucosid (50 Gew.-% Aktivsubstanz) mit einem mittleren Polymerisationsgrad von 1,2 -1,4. Die C Kettenverteilung im Alkylpolyglucosid ist wie folgt: C8 0 - 3 %, C10 0 - 4 %, C12 67 - 75 %, C1423-30%,C160-2%.

### Vergleichsbeispiel

In einem geeigneten Druckreaktor wurden 247,4 g (0,4 Mol) Glucopon® 215 CSUP (Alkylpolyglucoside auf Basis von C8 und C10 Fettalkoholen, Polymerisationsgrad 1,5), 400 g Wasser und 320 g (4,0 Mol) NaOH (50 %ig) vorgelegt und 2 Stunden auf 80°C erhitzt. Anschließend wurden bei 60°C und einem Druck von maximal 5 bar 202,0 g (4,0 Mol) Methylchlorid portionsweise zudosiert. Nach einer Dosierzeit von 7 Stunden war die Reaktion beendet. Zur Entfernung des bei der Reaktion entstandenen NaCl wurde das Produkt gefriergetrocknet und mehrfach mit Ethanol extrahiert. Die vereinigten Extrakte wurden im Rotationsverdampfer vom Ethanol befreit und in 70 % Wasser aufgenommen.

Das erhaltene klare Produkt besaß einen Polymerisationsgrad m von 1,5, einen Anteil von unter 5 Gew.-% Alkylether mit einer C-Kette von größer gleich 12 bezogen auf das Alkylethergemisch und einen Methylierungsgrad von n= 2,5.

### Solubilisierende Eigenschaften

Die solubilisierende Eigenschaft wurde wie folgt untersucht: Bestimmt wurde die Menge an Solubilisator, die erforderlich ist, um ein klare Lösung einer 1 Gew. %ige Lösung von Vitamin E (Copherol® 1250C, Fa. Cognis) bzw. eines UV Lichtschutzfilters (Neo Heliopan AV, INCI: Ethylhexyl Methoxycinnamate) in Wasser/Ethanol (75/25) zu erhalten. Je weniger Menge an Solubilisator erforderlich ist, um eine klare Lösung zu erhalten, desto bessere Solubilisierungseigenschaften hat eine Substanz. Tabelle 1 zeigt die Ergebnisse:

Beispiele 1, 2 und 3 sind erfindungsgemäß, Beispiel V1 ist das Vergleichsbeispiel. Angegeben sind jeweils die Mengen an Solubilisator in Gew.-%, die erforderlich sind, um eine klare Lösung zu erhalten.

| Beispiel Nr. | Methylierungsgrad (n) | 1 Gew.-% Copherol® 1250 C | 1 Gew.% Neo Heliopan |
|---|---|---|---|
| | | | |
| Herstellbeispiel 1 | 1,7 | 4% | 5% |
| Herstellbeispiel 2 | 2,5 | 5% | 5% |
| Herstellbeispiel 3 | 1,7 | 6% | 5 % |
| | | | |
| Vergleichsbeispiel | 2,5 | > 7% | >7% |

### Herstellung einer Nanoemulsion

Eine Mischung aus 4.9g APG-Methylether gemäß Herstellbeispiel 3, 0.7g Cetearyl Alcohol, 47.2g Oktan und 47.2g VE-Wasser wurden in ein Glasmantelgefäß gefüllt und unter Rühren (IKA Eurostar Digital, 600upm) auf eine Temperatur von 70-80°C erhitzt. Die Temperierung des Glasmantelgefässes erfolgt dabei mit einem programmierbaren Kryostaten (Haake P1-C35P). Während des Herstellvorganges wurden elektrische Leitfähigkeit und Temperatur mit einem Konduktometer (WTW ProfiLine LF 197-S) und einer Messonde (WTW TertraCon 325/S) gemessen. Diese Mischung zeigt bei Temperaturen oberhalb 65°C keine Leitfähigkeit, liegt also als W/O-Emulsion vor, die Phaseninversionstemperatur ist überschritten. Nun erfolgt eine Abkühlung auf Raumtemperatur, die Kühlrate betrug 0.7 °C/min. Bei Beginn der Inversion von W/O zu O/W beginnt die Leitfähigkeit zu steigen. Nach Erreichen von Raumtemperatur liegt eine bläuliche Nanoemulsion mit einer mittleren Partikelgröße um 200nm vor.

## Patentansprüche

1. Nanoemulsion, enthaltend eine Wasserphase und eine Ölphase, **dadurch gekennzeichnet, dass** sie mindestens einen Alkyl- und/oder Alkenylether von Alkyl- und/oder Alkenyl(poly)glycosiden der Formel (I-A)
(Gₘ-R¹)R²ₙ (I-A)
- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,0 bis 3,0, vorzugsweise 1,2 bis 1,8 darstellt, und
- n eine Zahl von 0,5 bis 5,0, vorzugsweise 1,4 bis 2,6 darstellt enthält.

2. Nanoemulsion nach Anspruch 1, **dadurch gekennzeichnet, dass** sie mindestens ein Alkyl- und/oder Alkenylethergemisch von Alkyl- und/oder Alkenyl(poly)glycosiden der Formel (I-B)
(Gₘ-R¹)R²ₙ (I-B)
- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,0 bis 3,0, vorzugsweise 1,2 bis 1,8 darstellt, und
- n eine Zahl von 0,5 bis 5,0, vorzugsweise 1,4 bis 2,6 darstellt wobei mindestens 50 Gew.-% der Alkyl- und/oder Alkenylether einen Rest R¹ mit einer C Kette größer gleich 12 enthalten,
enthält.

3. Nanoemulsion nach Anspruch 1 und/oder 2, **dadurch gekennzeichnet, dass** sie mindestens ein Alkyl- und/oder Alkenylethergemisch von Alkyl- und/oder Alkenylpolyglycosiden der Formel (I-C)
(Gₘ-R¹)R²ₙ (I-C)
- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten,
enthält.

4. Nanoemulsion nach einem der vorgenannten Ansprüche, **dadurch gekennzeichnet, dass** sie mindestens einen Co-Emulgator enthält.

5. Nanoemulsion nach einem der vorgenannten Ansprüche, **dadurch gekennzeichnet, dass** sie weniger als 10 Gew.-%, vorzugsweise weniger als 5 Gew.-%, insbesondere weniger als 2 Gew.-%, insbesondere weniger als 0,5 Gew.-% ethoxylierte Emulgatoren enthält.

6. Verfahren zur Herstellung von Nanoemulsionen nach mindestens einem der vorgenannten Ansprüche, **dadurch gekennzeichnet, dass** man entweder
- die Ölphase zusammen mit mindestens einem Alkyl- und/oder Alkenylether von Alkyl- und/oder Alkenyl(poly)glycosiden der Formel (I-A) und/oder (I-B) und/oder (I-C) und der Wasserphase auf eine Temperatur oberhalb der Phaseninversionstemperatur erhitzt und diese Mischung abkühlt oder
- die Ölphase zusammen mit mindestens einem Alkyl- und/oder Alkenylether von Alkyl- und/oder Alkenyl(poly)glycosiden der Formel (I-A) und/oder (I-B) und/oder (I-C) auf eine Temperatur oberhalb der Phaseninversionstemperatur erhitzt und diese durch Zugabe der nicht erhitzten Wasserphase abkühlt.

7. Verwendung von Nanoemulsionen nach einen der vorgenannten Ansprüche zur Herstellung von oder in kosmetischen und/oder pharmazeutischen Zubereitungen.

8. Verwendung von Alkyl- und/oder Alkenylethern von Alkyl- und/oder Alkenyl(poly)glycosiden der Formel (I-A)
(Gₘ-R¹)R²ₙ (I-A)
- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,0 bis 3,0, vorzugsweise 1,2 bis 1,8 darstellt, und
- n eine Zahl von 0,5 bis 5,0, vorzugsweise 1,4 bis 2,6 darstellt
und/oder von Alkyl- und/oder Alkenylethergemischen von Alkyl- und/oder Alkenyl(poly)glycosiden der Formel (I-B)
(Gₘ-R¹)R²ₙ (I-B)
- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,0 bis 3,0 vorzugsweise 1,2 bis 1,8 darstellt, und
- n eine Zahl von 0,5 bis 5,0, vorzugsweise 1,4 bis 2,6 darstellt wobei mindestens 50 Gew.-% der Alkyl- und/oder Alkenylether einen Rest R¹ mit einer C Kette größer gleich 12 enthalten,
zur Herstellung von Nanoemulsionen, insbesondere zur Herstellung von Nanoemulsionen nach dem PIT-Verfahren.

9. Alkyl- und/oder Alkenylethergemisch von Alkyl- und/oder Alkenylpolyglycosiden der Formel (I-C)
(Gₘ-R¹)R²ₙ (I-C)
- in der G für einen Zuckerrest mit 5 oder 6 C-Atomen steht,
- R¹ für einen C6 bis C22 Alkyl- und/oder Alkenylrest in Acetalbindung steht,
- R² für eine C1 bis C4 Alkyl- und/oder Alkenylgruppe in Etherbindung steht,
- m einen mittleren Wert von 1,2 bis 1,8 darstellt, und
- n eine Zahl von 1,4 bis 2,6 darstellt
wobei mindestens 50 Gew.-% der Alkyl- und/oder Alkenylether einen Rest R¹ mit einer C Kette größer gleich 12 enthalten.

10. Verfahren zur Herstellung von Alkyl- und/oder Alkenylethergemischen nach Anspruch 9, **dadurch gekennzeichnet, dass** man Alkyl- und/oder Alkenylpolyglycoside der Formel (II)
Gₘ-R¹ (II)
- in der G für einen Zuckerrest mit 5 oder 6 C-Atomen steht,
- R¹ für einen C6 bis C22 Alkyl- und/oder Alkenylrest in Acetalbindung steht,
- m einen mittleren Wert von 1,2 bis 1,8 darstellt, und
wobei mindestens 50 Gew.-% der Alkyl- und/oder Alkenylpolyglycoside einen Rest R¹ mit einer C Kette größer gleich 12 enthalten
mit Alkylierungsmittel der Formel (III) R²-X
- in der X eine nucleophile Abhangsgruppe darstellt, und
- R² für eine C1 bis C4 Alkyl- und/oder Alkenylgruppe steht,
umsetzt.

11. Verwendung von Alkyl- und/oder Alkenylethergemischen nach Anspruch 9 als Solublisatoren.

12. Verwendung von Alkyl- und/oder Alkenylethergemischen nach Anspruch 9 zur Herstellung von oder in kosmetischen und/oder pharmazeutischen Zubereitungen, insbesondere in Form von Nanoemulsionen.

13. Zubereitungen, enthaltend 0,1 bis 20 Gew.-% Alkyl- und/oder Alkenylethergemische nach Anspruch 9.

14. Zubereitungen enthaltend Alkyl- und/oder Alkenylethergemische nach Anspruch 9 und/oder eine Nanoemulsion nach einem der Ansprüche 1 bis 5 und weiterhin mindestens einen UV-Lichtschutzfilter und/oder mindestens Vitamin und/oder mindestens ein Parfümöl sowie Mischungen daraus.

15. Zubereitungen enthaltend Alkyl- und/oder Alkenylethergemische nach Anspruch 9 und weiterhin mindestens einen Ölkörper und/oder mindestens einen Co-Emulgator sowie Mischungen daraus.

## Claims

1. A nanoemulsion comprising a water phase and an oil phase, **characterized in that** it comprises at least one alkyl and/or alkenyl ether of alkyl and/or alkenyl (poly)glycosides of the formula (I-A)
(Gₘ-R¹)R²ₙ (I-A)
- in which G is a sugar radical having 5 or 6 carbon atoms,
- R¹ is a C6 to C22 alkyl and/or alkenyl radical in acetal bond,
- R² is a C1 to C4 alkyl and/or alkenyl group in ether bond,
- m is an average value from 1.0 to 3.0, preferably 1.2 to 1.8, and
- n is a number from 0.5 to 5.0, preferably 1.4 to 2.6.

2. The nanoemulsion as claimed in claim 1, **characterized in that** it comprises at least one alkyl and/or alkenyl ether mixture of alkyl and/or alkenyl (poly)glycosides of the formula (I-B)
(Gₘ-R¹)R²ₙ (I-B)
- in which G is a sugar radical having 5 or 6 carbon atoms,
- R¹ is a C6 to C22 alkyl and/or alkenyl radical in acetal bond,
- R² is a C1 to C4 alkyl and/or alkenyl group in ether bond,
- m is an average value from 1.0 to 3.0, preferably 1.2 to 1.8, and
- n is a number from 0.5 to 5.0, preferably 1.4 to 2.6,
where at least 50% by weight of the alkyl and/or alkenyl ethers comprise a radical R¹ with a carbon chain greater than or equal to 12.

3. The nanoemulsion as claimed in claim 1 and/or 2, **characterized in that** it comprises at least one alkyl and/or alkenyl ether mixture of alkyl and/or alkenyl polyglycosides of the formula (I-C)
(Gₘ-R¹)R²ₙ (I-C)
- in which G is a sugar radical having 5 or 6 carbon atoms,
- R¹ is a C6 to C22 alkyl and/or alkenyl radical in acetal bond,
- R² is a C1 to C4 alkyl and/or alkenyl group in ether bond,
- m is an average value from 1.2 to 1.8, and
- n is a number from 1.4 to 2.6,
where at least 50% by weight of the alkyl and/or alkenyl ethers comprise a radical R¹ with a carbon chain greater than or equal to 12.

4. The nanoemulsion as claimed in any one of the preceding claims, **characterized in that** it comprises at least one coemulsifier.

5. The nanoemulsion as claimed in any one of the preceding claims, **characterized in that** it comprises less than 10% by weight, preferably less than 5% by weight, in particular less than 2% by weight, in particular less than 0.5% by weight, of ethoxylated emulsifiers.

6. A method of preparing nanoemulsions according to at least one of the preceding claims, **characterized in that** either
- the oil phase is heated together with at least one alkyl and/or alkenyl ether of alkyl and/or alkenyl (poly)glycosides of the formula (I-A) and/or (I-B) and/or (I-C) and the water phase to a temperature above the phase inversion temperature and this mixture is cooled or
- the oil phase is heated together with at least one alkyl and/or alkenyl ether of alkyl and/or alkenyl (poly)glycosides of the formula (I-A) and/or (I-B) and/or (I-C) to a temperature above the phase inversion temperature, and this is cooled by adding the nonheated water phase.

7. The use of nanoemulsions as claimed in any one of the preceding claims for the preparation of or in cosmetic and/or pharmaceutical preparations.

8. The use of alkyl and/or alkenyl ethers of alkyl and/or alkenyl (poly)glycosides of the formula (I-A)
(Gₘ-R¹)R²ₙ (I-A)
- in which G is a sugar radical having 5 or 6 carbon atoms,
- R¹ is a C6 to C22 alkyl and/or alkenyl radical in acetal bond,
- R² is a C1 to C4 alkyl and/or alkenyl group in ether bond,
- m is an average value from 1.0 to 3.0, preferably 1.2 to 1.8, and
- n is a number from 0.5 to 5.0, preferably 1.4 to 2.6 and/or of alkyl and/or alkenyl ether mixtures of alkyl and/or alkenyl (poly)glycosides of the formula (I-B) (Gₘ-R¹)R²ₙ (I-B)
- in which G is a sugar radical having 5 or 6 carbon atoms,
- R¹ is a C6 to C22 alkyl and/or alkenyl radical in acetal bond,
- R² is a C1 to C4 alkyl and/or alkenyl group in ether bond,
- m is an average value from 1.0 to 3.0, preferably 1.2 to 1.8, and
- n is a number from 0.5 to 5.0, preferably 1.4 to 2.6,
where at least 50% by weight of the alkyl and/or alkenyl ethers comprise a radical R¹ with a carbon chain greater than or equal to 12,
for the preparation of nanoemulsions, in particular for the preparation of nanoemulsions according to the PIT method.

9. An alkyl and/or alkenyl ether mixture of alkyl and/or alkenyl polyglycosides of the formula (I-C)
(Gₘ-R¹)R²ₙ (I-C)
- in which G is a sugar radical having 5 or 6 carbon atoms,
- R¹ is a C6 to C22 alkyl and/or alkenyl radical in acetal bond,
- R² is a C1 to C4 alkyl and/or alkenyl group in ether bond,
- m is an average value from 1.2 to 1.8, and
- n is a number from 1.4 to 2.6,
where at least 50% by weight of the alkyl and/or alkenyl ethers comprise a radical R¹ with a carbon chain greater than or equal to 12.

10. A method of preparing alkyl and/or alkenyl ether mixtures as claimed in claim 9, **characterized in that** alkyl and/or alkenyl polyglycosides of the formula (II)
Gₘ-R¹ (II)
- in which G is a sugar radical having 5 or 6 carbon atoms,
- R¹ is a C6 to C22 alkyl and/or alkenyl radical in acetal bond,
- m is an average value from 1.2 to 1.8, and
where at least 50% by weight of the alkyl and/or alkenyl polyglycosides comprise a radical R¹ with a carbon chain greater than or equal to 12
are reacted with alkylating agents of the formula (III) R²-X
- in which X is a nucleophilic leaving group, and
- R² is a C1 to C4 alkyl and/or alkenyl group.

11. The use of alkyl and/or alkenyl ether mixtures as claimed in claim 9 as solubilizers.

12. The use of alkyl and/or alkenyl ether mixtures as claimed in claim 9 for the preparation of or in cosmetic and/or pharmaceutical preparations, in particular in the form of nanoemulsions.

13. A preparation comprising 0.1 to 20% by weight of alkyl and/or alkenyl ether mixtures as claimed in claim 9.

14. A preparation comprising alkyl and/or alkenyl ether mixtures as claimed in claim 9 and/or a nanoemulsion as claimed in any one of claims 1 to 5 and furthermore at least one UV photoprotective filter and/or at least vitamin and/or at least one perfume oil, and mixtures thereof.

15. A preparation comprising alkyl and/or alkenyl ether mixtures as claimed in claim 9 and furthermore at least one oil body and/or at least one coemulsifier, and mixtures thereof.

## Revendications

1. Nanoémulsion, contenant une phase aqueuse et une phase huileuse, **caractérisée en ce qu'**elle contient au moins un alkyléther et/ou un alcényléther d'alkyl(poly)glycosides et/ou d'alcényl(poly)glycosides de formule (I-A)
(Gₘ-R¹)R²ₙ (I-A)
- dans laquelle G représente un radical de sucre comprenant 5 ou 6 atomes de carbone,
- R¹ représente un radical C₆-C₂₂-alkyle et/ou C₆-C₂₂-alcényle en liaison acétal,
- R² représente un radical C₁-C₄-alkyle et/ou C₁-C₄-alcényle en liaison éther,
- m représente une valeur moyenne de 1,0 à 3,0, de préférence de 1,2 à 1,8, et
- n représente un nombre de 0,5 à 5,0, de préférence de 1,4 à 2,6.

2. Nanoémulsion selon la revendication 1, **caractérisée en ce qu'**elle contient au moins mélange d'alkyléthers et/ou d'alcényléthers d'alkyl(poly)glycosides et/ou d'alcényl(poly)glycosides de formule (I-B)
(Gₘ-R¹)R²ₙ (I-B)
- dans laquelle G représente un radical de sucre comprenant 5 ou 6 atomes de carbone,
- R¹ représente un radical C₆-C₂₂-alkyle et/ou C₆-C₂₂-alcényle en liaison acétal,
- R² représente un radical C₁-C₉-alkyle et/ou C₁-C₄-alcényle en liaison éther,
- m représente une valeur moyenne de 1,0 à 3,0, de préférence de 1,2 à 1,8, et
- n représente un nombre de 0,5 à 5,0, de préférence de 1,4 à 2,6,
où au moins 50% en poids des alkyléthers et/ou des alcényléthers contiennent un radical R¹ présentant une chaîne carbonée supérieure à 12.

3. Nanoémulsion selon la revendication 1 et/ou 2, **caractérisée en ce qu'**elle contient au moins mélange d'alkyléthers et/ou d'alcényléthers d'alkylpolyglycosides et/ou d'alcénylpolyglycosides de formule (I-C)
(Gₘ-R¹)R²ₙ (1-C)
- dans laquelle G représente un radical de sucre comprenant 5 ou 6 atomes de carbone,
- R¹ représente un radical C₆-C₂₂-alkyle et/ou C₆-C₂₂-alcényle en liaison acétal,
- R² représente un radical C₁-C₉-alkyle et/ou C₁-C₉-alcényle en liaison éther,
- m représente une valeur moyenne de 1,2 à 1,8, et
- n représente un nombre de 1,4 à 2,6 où au moins 50% en poids des alkyléthers et/ou des alcényléthers contiennent un radical R¹ présentant une chaîne carbonée supérieure à 12.

4. Nanoémulsion selon l'une quelconque des revendications susmentionnées, **caractérisée en ce qu'**elle contient au moins un co-émulsifiant.

5. Nanoémulsion selon l'une quelconque des revendications susmentionnées, **caractérisée en ce qu'**elle contient moins de 10% en poids, de préférence moins de 5% en poids, en particulier moins de 2% en poids, en particulier moins de 0,5% en poids d'émulsifiants éthoxylés.

6. Procédé pour la préparation de nanoémulsions selon au moins l'une quelconque des revendications susmentionnées, **caractérisé en ce que**
- soit on chauffe la phase huileuse ensemble avec au moins un alkyléther et/ou un alcényléther d'alkyl(poly)glycosides et/ou d'alcényl(poly)glycosides de formule (I-A) et/ou (I-B) et/ou (I-C) et la phase aqueuse à une température supérieure à la température d'inversion des phases et on refroidit ce mélange,
- soit on chauffe la phase huileuse ensemble avec au moins un alkyléther et/ou un alcényléther d'alkyl(poly)glycosides et/ou d'alcényl(poly)glycosides de formule (I-A) et/ou (I-B) et/ou (I-C) à une température supérieure à la température d'inversion des phases et on la refroidit par addition de la phase aqueuse non chauffée.

7. Utilisation de nanoémulsions selon l'une quelconque des revendications susmentionnées pour la préparation de ou dans des compositions cosmétiques et/ou pharmaceutiques.

8. Utilisation d'alkyléthers et/ou d'alcényléthers d'alkyl(poly)glycosides et/ou d'alcényl(poly)glycosides de formule (I-A)
(Gₘ-R¹)R²ₙ (I-A)
- dans laquelle G représente un radical de sucre comprenant 5 ou 6 atomes de carbone,
- R¹ représente un radical C₆-C₂₂-alkyle et/ou C₆-C₂₂-alcényle en liaison acétal,
- R² représente un radical C₁-C₄-alkyle et/ou C₁-C₄-alcényle en liaison éther,
- m représente une valeur moyenne de 1,0 à 3,0, de préférence de 1,2 à 1,8, et
- n représente un nombre de 0,5 à 5,0, de préférence de 1,4 à 2,6.
et/ou de mélanges d'alkyléthers et/ou d'alcényléthers d'alkyl(poly)glycosides et/ou d'alcényl(poly)glycosides de formule (I-B)
(Gₘ-R¹)R²ₙ (I-B)
- dans laquelle G représente un radical de sucre comprenant 5 ou 6 atomes de carbone,
- R¹ représente un radical C₆-C₂₂-alkyle et/ou C₆-C₂₂-alcényle en liaison acétal,
- R² représente un radical C₁-C₄-alkyle et/ou Ci-cor alcényle en liaison éther,
- m représente une valeur moyenne de 1,0 à 3,0, de préférence de 1,2 à 1,8, et
- n représente un nombre de 0,5 à 5,0, de préférence de 1,4 à 2,6.
où au moins 50% en poids des alkyléthers et/ou des alcényléthers contiennent un radical R¹ présentant une chaîne carbonée supérieure à 12,
pour la préparation de nanoémulsions, en particulier pour la préparation de nanoémulsions selon le procédé PIT.

9. Mélange d'alkyléthers et/ou d'alcényléthers d'alkylpolyglycosides et/ou d'alcénylpolyglycosides de formule (I-C)
(Gₘ-R¹)R²ₙ (I-C)
- dans laquelle G représente un radical de sucre comprenant 5 ou 6 atomes de carbone,
- R¹ représente un radical C₆-C₂₂-alkyle et/ou C₆-C₂₂-alcényle en liaison acétal,
- R² représente un radical C₁-C₄-alkyle et/ou C₁-C₄-alcényle en liaison éther,
- m représente une valeur moyenne de 1,2 à 1,8, et
- n représente un nombre de 1,4 à 2,6 où au moins 50% en poids des alkyléthers et/ou des alcényléthers contiennent un radical R¹ présentant une chaîne carbonée supérieure à 12.

10. Procédé pour la préparation de mélanges d'alkyléthers et/ou d'alcényléthers selon la revendication 9, **caractérisé en ce qu'**on transforme des alkylpolyglycosides et/ou des alcénylpolyglycosides de formule (II)
Gₘ-R¹ (II)
- dans laquelle G représente un radical de sucre comprenant 5 ou 6 atomes de carbone,
- R¹ représente un radical C₆-C₂₂-alkyle et/ou C₆-C₂₂-alcényle en liaison acétal,
- m représente une valeur moyenne de 1,2 à 1,8, et où au moins 50% en poids des alkylpolyglycosides et/ou des alcénylpolyglycosides contiennent un radical R¹ présentant une chaîne carbonée supérieure à 12 avec un agent d'alkylation de formule (III) R²-X
- dans laquelle X représente un groupe partant nucléophile, et
- R² représente un radical C₁-C₄-alkyle et/ou C₁-C₉-alcényle.

11. Utilisation de mélanges d'alkyléthers et/ou d'alcényléthers selon la revendication 9 comme agents de solubilisation.

12. Utilisation de mélanges d'alkyléthers et/ou d'alcényléthers selon la revendication 9 pour la préparation de ou dans des compositions cosmétiques et/ou pharmaceutiques, en particulier sous forme de nanoémulsions.

13. Compositions, contenant 0,1 à 20% en poids de mélanges d'alkyléthers et/ou d'alcényléthers selon la revendication 9.

14. Compositions contenant des mélanges d'alkyléthers et/ou d'alcényléthers selon la revendication 9 et/ou une nanoémulsion selon l'une quelconque des revendications 1 à 5 et en outre au moins un filtre de protection contre la lumière UV et/ou au moins une vitamine et/ou au moins une huile parfumée, ainsi que leurs mélanges.

15. Compositions contenant des mélanges d'alkyléthers et/ou d'alcényléthers selon la revendication 9 et en outre au moins un corps huileux et/ou au moins co-émulsifiant ainsi que leurs mélanges.
